# EUROPEAN PATENT APPLICATION

(11) **EP 1 035 207 A1**
(43) Date of publication of application: **13.09.2000**
(21) Application number: 99104664.0
(22) Date of filing: 09.03.1999
(51) Int. Cl.: C12N 15/53, C12N 15/11, C12N 15/85, C12N 9/02, C12N 5/10, C12Q 1/02, C07K 16/40, A61K 39/395, A61K 38/44, A01K 67/027, G01N 33/50, G01N 33/53

(54) **cDNA molecules of the members of gene family encoding human fatty acid desaturases and their use in diagnosis and therapy**

(71) Applicant: MultiGene Biotech GmbH, 97074 Würzburg (DE)
(72) Inventor: Weber, Bernhard H.F., 97218 Gerbrunn (DE); Marquardt, Andreas, 97218 Gerbrunn (DE)
(74) Representative: Schmidt, Werner, Dr.

(57) **Abstract**

The present invention relates to the cloning and sequencing of the cDNA molecules of three members of a gene family encoding three human fatty acid desaturases, fatty acid desaturase-1 (FADS 1), fatty acid desaturase-2 (FADS2) and fatty acid desaturase-3 (FADS3). The invention also relates to diagnostic methods of screening for and detection of FADS1, FADS2, FADS3 and gene therapy utilizing recombinant DNA as well as the generation of animal models (knock-in, knock-out, transgenic animals), anti-FADS1, anti-FADS2, anti-FADS3 antibodies and use in screenings for modulating drugs.

### Annex to the application documents - subsquently filed sequences listing

SEQUENCE LISTING

(1) GENERAL INFORMATION:

(i) APPLICANT:
(A) NAME: MultiGen Biotech GmbH
(B) STREET: Am Hubland
(C) CITY: Wuerzburg
(D) STATE: -
(E) COUNTRY: Germany
(F) POSTAL CODE (ZIP): 97074
(G) TELEPHONE: 0931-7058-4340
(H) TELEFAX: 0931-7058-4355
(I) TELEX: -

(ii) TITLE OF INVENTION: cDNA molecules of the members of a gene family
encoding human fatty acid desaturases and their use in
diagnosis and therapy

(iii) NUMBER OF SEQUENCES: 22

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 444 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION:1..444

(2) INFORMATION FOR SEQ ID NO: 1:

(i) SEQUENCE CHARACTERISTICS:
(A) LENGTH: 4204 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..4204

(xi) SEQUENCE DESCRIPTION: SEQ ID NO: 1:

CACTCCTGGA GCCCGCGGAC CCCGAGCACG CGCCTGACAG CCCCTGCTGG CCCGGCGCGC 60

GGCGTCGCCA GGCCAGCTAT GGCCCCCGAC CCGGTGGCCG CCGAGACCGC
GGCTCAGGGA 120

CCTACCCCGC GCTACTTCAC CTGGGACGAG GTGGCCCAGC GCTCAGGGTG
CGAGGAGCGG 180

TGGCTAGTGA TCGACCGTAA GGTGTACAAC ATCAGCGAGT TCACCCGCCG
GCATCCAGGG 240

GGCTCCCGGG TCATCAGCCA CTACGCCGGG CAGGATGCCA CGGATCCCTT
TGTGGCCTTC 300

CACATCAACA AGGGCCTTGT GAAGAAGTAT ATGAACTCTC TCCTGATTGG
AGAACTGTCT 360

CCAGAGCAGC CCAGCTTTGA GCCCACCAAG AATAAAGAGC TGACAGATGA
GTTCCGGGAG 420

CTGCGGGCCA CAGTGGAGCG GATGGGGCTC ATGAAGGCCA ACCATGTCTT
CTTCCTGCTG 480

TACCTGCTGC ACATCTTGCT GCTGGATGGT GCAGCCTGGC TCACCCTTTG
GGTCTTTGGG 540

ACGTCCTTTT TGCCCTTCCT CCTCTGTGCG GTGCTGCTCA GTGCAGTTCA
GGCCCAGGCT 600

GGCTGGCTGC AGCATGACTT TGGGCACCTG TCGGTCTTCA GCACCTCAAA
GTGGAACCAT 660

CTGCTACATC ATTTTGTGAT TGGCCACCTG AAGGGGGCCC CCGCCAGTTG
GTGGAACCAC 720

ATGCACTTCC AGCACCATGC CAAGCCCAAC TGCTTCCGCA AAGACCCAGA
CATCAACATG 780

CATCCCTTCT TCTTTGCCTT GGGGAAGATC CTCTCTGTGG AGCTTGGGAA
ACAGAAGAAA 840

AAATATATGC CGTACAACCA CCAGCACAAA TACTTCTTCC TAATTGGGCC
CCCAGCCTTG 900

CTGCCTCTCT ACTTCCAGTG GTATATTTTC TATTTTGTTA TCCAGCGAAA
GAAGTGGGTG 960

GACTTGGCCT GGATGATTAC CTTCTACGTC CGCTTCTTCC TCACTTATGT
GCCACTATTG 1020

GGGCTGAAAG CCTTCCTGGG CCTTTTCTTC ATAGTCAGGT TCCTGGAAAG
CAACTGGTTT 1080

GTGTGGGTGA CACAGATGAA CCATATTCCC ATGCACATTG ATCATGACCG
GAACATGGAC 1140

TGGGTTTCCA CCCAGCTCCA GGCCACATGC AATGTCCACA AGTCTGCCTT
CAATGACTGG 1200

TTCAGTGGAC ACCTCAACTT CCAGATTGAG CACCATCTTT TTCCCACGAT
GCCTCGACAC 1260

AATTACCACA AAGTGGCTCC CCTGGTGCAG TCCTTGTGTG CCAAGCATGG
CATAGAGTAC     1320

CAGTCCAAGC CCCTGCTGTC AGCCTTCGCC GACATCATCC ACTCACTAAA
GGAGTCAGGG     1380

CAGCTCTGGC TAGATGCCTA TCTTCACCAA TAACAACAGC CACCCTGCCC
AGTCTGGAAG     1440

AAGAGGAGGA AGACTCTGGA GCCAAGGCAG AGGGGAGCTT GAGGGACAAT
GCCACTATAG     1500

TTTAATACTC AGAGGGGGTT GGGTTTGGGG ACATAAAGCC TCTGACTCAA
ACTCCTCCCT     1560

TTTATCTTCT AGCCACAGTT CTAAGACCCA AAGTGGGGGG TGGACACAGA
AGTCCCTAGG     1620

AGGGAAGGAG CTGTTGGGGC AGGGGTGTAA ATTATTTCCT TTTTCTAGTT
TGGCACATGC     1680

AGGTAGTTGG TGAACAGAGA GAACCAGGAG GGTAACAGAA GAGGAGGGAC
CTACTGAACC     1740

CAGAGTCAGG AAGAGATTTA ACACTAAAAT TCCACTCATG CCGGGCGTGG
TGCACGCGCC     1800

TGTAATCCCA GCTACCCAGG AGGCTGAGGC AGGAGAATCG CTTGAACCGG
GGAGGTGGAG     1860

GTTGCAGTGA GCTGAGATCA CGCCATTGTA CTCCAGCCTG GGCGACAGAG
CAAGACTCCA     1920

TTTCAAAAAA AAAAAAAAAA AAAAAAATCC ACTCATATAA AAGGTGAGCT
CAGCTCACTG     1980

GTCCATTTCT CAGTGGCTTC TCCATCCTCA TTTGCAAACC TCAGAGGGAT
AAGGCAGTTG     2040

AACCTGATGA GCAAGAATTA TAACAGCAAG GAAACATTAA TGCTTAGAAT
TCTGAGATCC     2100

AGCACAACTC AGTCTGTGGG AGCTCAGCTC GCTGCCCAGG GATAGGTATG
ACCTATGTCT     2160

GCCTTAGGCT GCTGGGAGAT GCCATTCTCC AGTTTCAGAA GCAGGCAGGG
CAAAGGTCAA     2220

GACTGTGGTA TTGGGGTCTT TTGGCTCTGA AGGATCCTGG AACCACTGAT
TTTGGTTTAT     2280

TCCCTCCAGG GTCTAAAGAG AACAAGAGGT GCTAGCTCTT ACCAAAACAG
ATGGTAGAGA     2340

GAGTTGCTGG CTATTTAAAA AGCTCTTTCA TCTTTTAATT CACCTCTTCT
TTTCACCTCT     2400

TTAACCACTC CTCAGGAACA GAACACTTCT AGGACTGGGG GTCTTTTAGC
TCCATAAGCA     2460

AGTGAGCAGA TGGGACAAGT TAGTCTTTTC TCCCTAGAAA CAAAGGGGAT
GCCCAGTGGT     2520

TTCCCTTTGC TTCCCAACCT AAAATTTCAA GTTTAATAAA ATAGCAATTA
GCAGAAGTGA 2580

CCAAATTGGG AGATAATTAT CAGTCATGAG GAAAGACACA GATTTCGGTC
ATAAAGAATG 2640

TAAGGGCTAT AAGTAGAAAC TTTCTATAAC CTAAATGATG TTATAGAATT
ATTTTTGAGC 2700

AGGAGCAGAA AGATTAAATA TGATCACTTC ATACTTCTAA ATCAGAAATA
GGAAGATTAA 2760

AACCACAGAA CAGTTTGTGA TTTCTATTGC TGGTAGCTAG GTATCTTACT
CTGTCCACTC 2820

TTGTTCAAGT ATCTAACTCT TCTGGAAACC AAATAGGCTT TAGAAGAGAT
TATCCTATAT 2880

TCCTATCAGT ATAATACTAA AATGTAACTT TTTAATCATC TGGTTTTTAA
AAGATAAACA 2940

GTTTAGCCCA TCTCTCCAGA GAGCAAACAT AGGAATATGA CTCAGGAGCC
TCCTAGGGCT 3000

TATCATCAGC CCTCACACCC GCTTCCCCCT CCAACCCACA GCCTTTGCTT
CCAGGTGGCA 3060

GGATTACTAC TTTGCCTCTT CAGCAGCATC TACTCTAGGC ATATTGATCA
TTTTAGACAC 3120

TGGGAGAAGA GAACCTCAAA CTAGGAGGAA AAGACAGAGC CTCCACTTAG
TTTTGGGAGG 3180

GGATGGCAGA CAGTCAAGGA GATGAGCGTC CTAAGGCATG TTGGGATAGG
GTCAGATGCA 3240

CCACCCATGG AGAGGTTTGT CAACACAAAG ACATGGAAGG TTAGAGGTTT
GTCAACAAAA 3300

AGACATGGAA GGTTAGGTTT GTCAACACAA AGACATGGAA GATTAGAGGT
TTGTCAACAC 3360

AAAGATACAG GAAGAATGGG CTGCAGAAGA TTTAGATGTT TTCCATTTGG
GCACATTTTA 3420

CTTAGCTGGA GAACTAGGTT TAAAACAGCC TGGGTAGGAA AATTAGAAGC
AAGCTGGATG 3480

CAGTGGCTCA TGCCTGTAAT CCCAACACTT TTGGGAGGTC CAGGCAGGAG
GATCACTTGG 3540

GCCCAGGAGG TCAAGCCTGC AGCGAGCTGA GATCACACCA CTGCACTCCA
GCCTGGGGTG 3600

ATAGAACAAG ACCCTGTCTC AAAAAAAAAA AAAAACAACA AAAACTTAGA
ATTGAGGAGT 3660

TGTACCTCCA TTGGCTTCCT CACTCCAAAA TAGGTGCTGA TCCTTCCTAT
TCCTATTCTT 3720

TGCCACCTTT TGGGTGTGGT GTCACCAGCC TGTTTAGCCA AGTAGCTTTG
GGCATAGGCT 3780

GCCCAATCTG AGCAAACACC AGTGAGGCTC TATTGAGCAA GACCAAGTCC
TCAAAGCACC    3840

TGAACCACTG TGGCCTTCTC AGCCTACAGC AGTGTGGTCT CTTACATGGC
CACAAAGGGA    3900

CACACAGTGA CAAAAGGCTC GGAATGTTAC AATGGTAAAA TGAGTGATCT
CAAATCCACT    3960

GACAGATATA AAATAGGCTT AGAGAGGAAA AGCTGCCTCT GGTCAAGTAG
ATCATGGCAG    4020

CATGAATTCC AACTCACTTT TTTACGAACT CCAACTTCTA TGTTTATCTT
TGTTACTTTC    4080

ACTTTTTTAC AACCTGCAGA GGCATTTTTT AAATCAGGCC CAATATCAGT
ATTCTTTTTG    4140

TGTGTGCCAA TTTTGTTATC ACATCCCTAT GAAGTTGAAA AATAAAGTTA
ATTTTGACCA    4200

AAAG                                                                                                    4204

(2) INFORMATION FOR SEQ ID NO: 2:

(i) SEQUENCE CHARACTERISTICS:
(A) LENGTH: 4089 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..4089

(xi) SEQUENCE DESCRIPTION: SEQ ID NO: 2:

CGTCACAGTC GGCAGGCAGC ATGGGGAAGG GAGGGAACCA GGGCGAGGGG
GCCGCCGAGC 60

GCGAGGTGTC GGTGCCCACC TTCAGCTGGG AGGAGATTCA GAAGCATAAC
CTGCGCACCG 120

ACAGGTGGCT GGTCATTGAC CGCAAGGTTT ACAACATCAC CAAATGGTCC
ATCCAGCACC 180

CGGGGGGCCA GCGGGTCATC GGGCACTACG CTGGAGAAGA TGCAACGGAT
GCCTTCCGCG 240

CCTTCCACCC TGACCTGGAA TTCGTGGGCA AGTTCTTGAA ACCCCTGCTG
ATTGGTGAAC 300

TGGCCCCGGA GGAGCCCAGC CAGGACCACG GCAAGAACTC AAAGATCACT
GAGGACTTCC 360

GGGCCCTGAG GAAGACGGCT GAGGACATGA ACCTGTTCAA GACCAACCAC
GTGTTCTTCC 420

TCCTCCTCCT GGCCCACATC ATCGCCCTGG AGAGCATTGC ATGGTTCACT
GTCTTTTACT 480

TTGGCAATGG CTGGATTCCT ACCCTCATCA CGGCCTTTGT CCTTGCTACC
TCTCAGGCCC 540

AAGCTGGATG GCTGCAACAT GATTATGGCC ACCTGTCTGT CTACAGAAAA
CCCAAGTGGA 600

ACCACCTTGT CCACAAATTC GTCATTGGCC ACTTAAAGGG TGCCTCTGCC
AACTGGTGGA 660

ATCATCGCCA CTTCCAGCAC CACGCCAAGC CTAACATCTT CCACAAGGAT
CCCGATGTGA 720

ACATGCTGCA CGTGTTTGTT CTGGGCGAAT GGCAGCCCAT CGAGTACGGC
AAGAAGAAGC 780

TGAAATACCT GCCCTACAAT CACCAGCACG AATACTTCTT CCTGATTGGG
CCGCCGCTGC 840

TCATCCCCAT GTATTTCCAG TACCAGATCA TCATGACCAT GATCGTCCAT
AAGAACTGGG 900

TGGACCTGGC CTGGGCCGTC AGCTACTACA TCCGGTTCTT CATCACCTAC
ATCCCTTTCT 960

ACGGCATCCT GGGAGCCCTC CTTTTCCTCA ACTTCATCAG GTTCCTGGAG
AGCCACTGGT 1020

TTGTGTGGGT CACACAGATG AATCACATCG TCATGGAGAT TGACCAGGAG
GCCTACCGTG 1080

ACTGGTTCAG TAGCCAGCTG ACAGCCACCT GCAACGTGGA GCAGTCCTTC
TTCAACGACT 1140

GGTTCAGTGG ACACCTTAAC TTCCAGATTG AGCACCACCT CTTCCCCACC
ATGCCCCGGC 1200

ACAACTTACA CAAGATCGCC CCGCTGGTGA AGTCTCTATG TGCCAAGCAT
GGCATTGAAT 1260

ACCAGGAGAA GCCGCTACTG AGGGCCCTGC TGGACATCAT CAGGTCCCTG
AAGAAGTCTG 1320

GGAAGCTGTG GCTGGACGCC TACCTTCACA AATGAAGCCA CAGCCCCCGG
GACACCGTGG 1380

GGAAGGGGTG CAGGTGGGGT GATGGCCAGA GGAATGATGG GCTTTTGTTC
TGAGGGGTGT 1440

CCGAGAGGCT GGTGTATGCA CTGCTCACGG ACCCCATGTT GGATCTTTCT
CCCTTTCTCC 1500

TCTCCTTTTT CTCTTCACAT CTCCCCCATA GCACCCTGCC CTCATGGGAC
CTGCCCTCCC 1560

TCAGCCGTCA GCCATCAGCC ATGGCCCTCC CAGTGCCTCC TAGCCCCTTC
TTCCAAGGAG 1620

CAGAGAGGTG GCCACCGGGG GTGGCTCTGT CCTACCTCCA CTCTCTGCCC
CTAAAGATGG 1680

GAGGAGACCA GCGGTCCATG GGTCTGGCCT GTGAGTCTCC CCTTGCAGCC
TGGTCACTAG 1740

GCATCACCCC CGCTTTGGTT CTTCAGATGC TCTTGGGGTT CATAGGGGCA
GGTCCTAGTC 1800

GGGCAGGGCC CCTGACCCTC CCGGCCTGGC TTCACTCTCC CTGACGGCTG
CCATTGGTCC 1860

ACCCTTTCAT AGAGAGGCCT GCTTTGTTAC AAAGCTCGGG TCTCCCTCCT
GCAGCTCGGT 1920

TAAGTACCCG AGGCCTCTCT TAAGATGTCC AGGGCCCCAG GCCCGCGGGC
ACAGCCAGCC 1980

CAAACCTTGG GCCCTGGAAG AGTCCTCCAC CCCATCACTA GAGTGCTCTG
ACCCTGGGCT 2040

TTCACGGGCC CCATTCCACC GCCTCCCCAA CTTGAGCCTG TGACCTTGGG
ACCAAAGGGG 2100

GAGTCCCTCG TCTCTTGTGA CTCAGCAGAG GCAGTGGCCA CGTTCAGGGA
GGGGCCGGCT 2160

GGCCTGGAGG CTCAGCCCAC CCTCCAGCTT TTCCTCAGGG TGTCCTGAGG
TCCAAGATTC     2220

TGGAGCAATC TGACCCTTCT CCAAAGGCTC TGTTATCAGC TGGGCAGTGC
CAGCCAATCC     2280

CTGGCCATTT GGCCCCAGGG GACGTGGGCC CTGCAGGCTG CAGGAGGGCA
CTGGAGCTGG     2340

GAGGTCTCGT CCCAGCCCTC CCCATCTCGG GGCTGCTGTG TGGACGGCGC
TGCCTCAGGC     2400

ACTCTCCTGT CTGAACCTGC CCTTACTGTG TTTAACCTGT TGCTCCAGGA
TGCATTCTGA     2460

TAGGAGGGGG CGGCAGGGCT GGGCCTTGTG ACAATCTGCC TTTCACCACA
TGGCCTTGCC     2520

TCGGTGGCCC TGACTGTCAG GGAGGGCCAG GGAGGCAGAG CGGGAGGGAG
TCTCAGGAGG     2580

AGGCTGCCCT GAGGGGCTGG GGAGGGGGTA CCTCATGAGG ACCAGGGTGG
AGCTGAGAAG     2640

AGGAGGAGGT GGGGGCTGGA GGTGCTGGTA GCTGAGGGGA CGGGCAAGTG
AGAGGGGAGG     2700

GAGGGAAGTC CTGGGAGGAT CCTGAGCTGC TGTTGCAGTC TAACCCACTA
ATCAGTTCTT     2760

AGATTCAGGG GAAGGGCAGG CACCAACAAC TCAGAATGGG GGCTTTCGGG
GAGGGCGCCT     2820

AGTCCCCCCA GCTCTAAGCA GCCAGGAGGG ACCTGCATCT AAGCATCTGG
GTTGCCATGG     2880

CAATGGCATG CCCCCCAGCT ACTGTATGCC CCCGACCCCC GCAGAGGCAG
AATGAACCCA     2940

TAGGGAGCTG ATCGTAATGT TTATCATGTT ACTTCCCCAC CCCTACATTT
TTTGAAATAA     3000

AATAAGGAAT TTTATTCTCA CTTCCTGTGT TTCCTGCACG CCAATGCCAG
GCCATGGTAT     3060

TGGGTGATAG ATGAGGCCCT TCTAGCTGGG CCTGGGCACC AGGAGGGGTC
CCCATGCTTG     3120

CATCTCTCTG TATCCCCTCC CTCCCCTGTG GCCATCCCAC CCGCCTCTCC
CTGCTGCCTC     3180

TGAAATTCAT TCTGGGGCCC GGAACTTGGT GGAAATGACC CAAAAACATT
GGCCCATCTT     3240

CCTCCTCTCA GCAGCCGACC CCAGCCCAAT TCTAAAACAG GGCTGAGAGC
CACCTCTCAG     3300

CAGCTGACCC CTACCCAAGG AGGGTGGCAT GGAGGGGCTT GCAGAGACTC
TTCCTAACAT     3360

CCTCCCCCCC CAGCTGTCTC CCCAAGTGCA ATCTGCCCTC CCATCCCTGG
GCCAGCCAGC     3420

TTCCACAGAG CGCCAGGCCA AACAGAATTC CTGGCCTCCT TGGAAGGGGC
TGGAGAAGGC     3480

CGGGAGCAGT GGCTCACGCC TGTAATCCCA GCACTTTGGG AGGCTGAGGC
GGGCAGATCA     3540

CAAAGTCAAG AGATTGAGAC CATCCTGGCC AACATGGTGA AACCCCGTCT
CTACTAAAAA     3600

TACAAAAATT AGGCCGGGTG CGGTGGCTCA CGCCTGTAAT CCCAGCACTT
TGGGAGGCCG     3660

AGGCGGGCAG ATCACGAGGT CAGGAGATCA AGACCATCCT GGCTAACACG
GTGAAACCCC     3720

GTCTCTACTA AAAATACAAA AAATTAGCTG GGCGAGGTGG CGGGTGCCTG
TAGTCCCAGC     3780

TACGTGGGAG GCTGAGGCAA GAGAATGGCG TGAACCCCGG CGGGGCAGAG
CCTGCAGAGA     3840

GCTGAGATCA CACCACTGTA CTCCAGCCTG GGCGACAGCG AGACTCCGTC
TCAAAAAAAA     3900

AAAAAAAAAA AATTAGCTGG GCATGGTGGT GCGTGCCTGC AGTCCCAGCT
ACTCAGGAGG     3960

CTGAGACGGG AGAATCGCTT GAACCTGGGA GGCAGAGGTT GCAGTGAGCC
AAGATCGCTC     4020

ACTCCAGCCT AGCGACAGAG TGAGACTCCA TCTCAAATAA ATAAATAAAT
TAATTAATTA     4080

AATTAAATT                                                    4089

(2) INFORMATION FOR SEQ ID NO: 3:

(i) SEQUENCE CHARACTERISTICS:
(A) LENGTH: 1757 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..1757

(xi) SEQUENCE DESCRIPTION: SEQ ID NO: 3:

GGCCGCGGCG GCAGGGCGGG GCCGGAGCAG CGGGCGGCGG CGGAGGCGGC
GCCCGGGAGC 60

GCTCTTCGCT TCCCTCGGGG TCTTGCTCGG ACCTCGGCCA CCGCCTGGGA
TCCCCAGGAC 120

TCGTGCGTGC AGCATGGGCG GCGTCGGGGA GCCGGGACCG CGGGAGGGAC
CCGCGCAGCC 180

GGGGGCACCG CTGCCCACCT TCTGCTGGGA GCAGATCCGC GCGCACGACC
AGCCCGGCGA 240

CAAGTGGCTG GTCATCGAGC GCCGCGTCTA CGACATCAGC CGCTGGGCAC
AGCGGCACCC 300

AGGGGGCAGC CGCCTCATCG GCCACCACGG CGCTGAGGAC GCCACGGATG
CCTTCCGTGC 360

CTTCCATCAA GATCTCAATT TTGTGCGCAA GTTCCTACAG CCCCTGTTGA
TTGGAGAGCT 420

GGCTCCGGAA GAACCCAGCC AGGATGGACC CCTGAATGCG CAGCTGGTCG
AGGACTTCCG 480

AGCCCTGCAC CAGGCAGCCG AGGACATGAA GCTGTTTGAT GCCAGTCCCA
CCTTCTTTGC 540

TTTCCTACTG GGCCACATCC TGGCCATGGA GGTGCTGGCC TGGCTCCTTA
TCTACCTCCT 600

GGGTCCTGGC TGGGTGCCCA GTGCCCTGGC CGCCTTCATC CTGGCCATCT
CTCAGGCTCA 660

GTCCTGGTGT CTGCAGCATG ACCTGGGCCA TGCCTCCATC TTCAAGAAGT
CCTGGTGGAA 720

CCACGTGGCC CAGAAGTTCG TGATGGGGCA GCTAAAGGGC TTCTCCGCCC
ACTGGTGGAA 780

CTTCCGCCAC TTCCAGCACC ACGCCAAGCC CAACATCTTC CACAAAGACC
CAGACGTGAC 840

GGTGGCGCCC GTCTTCCTCC TGGGGGAGTC ATCCGTCGAG TATGGCAAGA
AGAAACGCAG 900

ATACCTACCC TACAACCAGC AGCACCTGTA CTTCTTCCTG ATCGGCCCGC
CGCTGCTCAC     960

CCTGGTGAAC TTTGAAGTGG AAAATCTGGC GTACATGCTG GTGTGCATGC
AGTGGGCGGA     1020

TTTGCTCTGG GCCGCCAGCT TCTATGCCCG CTTCTTCTTA TCCTACCTCC
CCTTCTACGG     1080

CGTCCCTGGG GTGCTGCTCT TCTTTGTTGC TGTCAGGGTC CTGGAAAGCC
ACTGGTTCGT     1140

GTGGATCACA CAGATGAACC ACATCCCCAA GGAGATCGGC CACGAGAAGC
ACCGGGACTG     1200

GGTCAGCTCT CAGCTGGCAG CCACCTGCAA CGTGGAGCCC TCACTTTTCA
CCAACTGGTT     1260

CAGCGGGCAC CTCAACTTCC AGATCGAGCA CCACCTCTTC CCCAGGATGC
CGAGACACAA     1320

CTACAGCCGG GTGGCCCCGC TGGTCAAGTC GCTGTGTGCC AAGCACGGCC
TCAGCTACGA     1380

AGTGAAGCCC TTCCTCACCG CGCTGGTGGA CATCGTCAGG TCCCTGAAGA
AGTCTGGTGA     1440

CATCTGGCTG GACGCCTACC TCCATCAGTG AAGGCAACAC CCAGGCGGGC
AGAGAAGGGC     1500

TCAGGGCACC AGCAACCAAG CCAGCCCCGG CGGGATCGAT ACCCCCACCC
CTCCACTGGC     1560

CAGCCTGGGG GTGCCCTGCC TGCCCTCCTG GTACTGTTGT CTTCCCCTCG
GCCCCCTCAC     1620

ATGTGTATTC AGCAGCCCTA TGGCCTTGGC TCTGGGCCTG ATGGGACAGG
GGTAGAGGGA     1680

AGGTGAGCAT AGCACATTTT CCTAGAGCGA GAATTGGGGG AAAGCTGTTA
TTTTTATATT     1740

AAAATACATT CAGATGT                                                                                    1757

(2) INFORMATION FOR SEQ ID NO: 4:

(i) SEQUENCE CHARACTERISTICS:
(A) LENGTH: 444 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION:1..444

(xi) SEQUENCE DESCRIPTION: SEQ ID NO: 4:

Met Ala Pro Asp Pro Val Ala Ala Glu Thr Ala Ala Gln Gly Pro Thr
1               5                   10                  15

Pro Arg Tyr Phe Thr Trp Asp Glu Val Ala Gln Arg Ser Gly Cys Glu
            20                  25                  30

Glu Arg Trp Leu Val Ile Asp Arg Lys Val Tyr Asn Ile Ser Glu Phe
        35                  40                  45

Thr Arg Arg His Pro Gly Gly Ser Arg Val Ile Ser His Tyr Ala Gly
    50                  55                  60

Gln Asp Ala Thr Asp Pro Phe Val Ala Phe His Ile Asn Lys Gly Leu
65                  70                  75                  80

Val Lys Lys Tyr Met Asn Ser Leu Leu Ile Gly Glu Leu Ser Pro Glu
                85                  90                  95

Gln Pro Ser Phe Glu Pro Thr Lys Asn Lys Glu Leu Thr Asp Glu Phe
            100                 105                 110

Arg Glu Leu Arg Ala Thr Val Glu Arg Met Gly Leu Met Lys Ala Asn
        115                 120                 125

His Val Phe Phe Leu Leu Tyr Leu Leu His Ile Leu Leu Leu Asp Gly
    130                 135                 140

Ala Ala Trp Leu Thr Leu Trp Val Phe Gly Thr Ser Phe Leu Pro Phe
145                 150                 155                 160

Leu Leu Cys Ala Val Leu Leu Ser Ala Val Gln Ala Gln Ala Gly Trp
                165                 170                 175

Leu Gln His Asp Phe Gly His Leu Ser Val Phe Ser Thr Ser Lys Trp
            180                 185                 190

Asn His Leu Leu His His Phe Val Ile Gly His Leu Lys Gly Ala Pro
        195                 200                 205

Ala Ser Trp Trp Asn His Met His Phe Gln His His Ala Lys Pro Asn
    210                 215                 220

Cys Phe Arg Lys Asp Pro Asp Ile Asn Met His Pro Phe Phe Phe Ala
225                 230                 235                 240

Leu Gly Lys Ile Leu Ser Val Glu Leu Gly Lys Gln Lys Lys Lys Tyr
                245                 250                 255

Met Pro Tyr Asn His Gln His Lys Tyr Phe Phe Leu Ile Gly Pro Pro
            260                 265                 270

Ala Leu Leu Pro Leu Tyr Phe Gln Trp Tyr Ile Phe Tyr Phe Val Ile
        275                 280                 285

Gln Arg Lys Lys Trp Val Asp Leu Ala Trp Met Ile Thr Phe Tyr Val
    290                 295                 300

Arg Phe Phe Leu Thr Tyr Val Pro Leu Leu Gly Leu Lys Ala Phe Leu
305                 310                 315                 320

Gly Leu Phe Phe Ile Val Arg Phe Leu Glu Ser Asn Trp Phe Val Trp
                325                 330                 335

Val Thr Gln Met Asn His Ile Pro Met His Ile Asp His Asp Arg Asn
            340                 345                 350

Met Asp Trp Val Ser Thr Gln Leu Gln Ala Thr Cys Asn Val His Lys
        355                 360                 365

Ser Ala Phe Asn Asp Trp Phe Ser Gly His Leu Asn Phe Gln Ile Glu
    370                 375                 380

His His Leu Phe Pro Thr Met Pro Arg His Asn Tyr His Lys Val Ala
385                 390                 395                 400

Pro Leu Val Gln Ser Leu Cys Ala Lys His Gly Ile Glu Tyr Gln Ser
                405                 410                 415

Lys Pro Leu Leu Ser Ala Phe Ala Asp Ile Ile His Ser Leu Lys Glu
            420                 425                 430

Ser Gly Gln Leu Trp Leu Asp Ala Tyr Leu His Gln
        435                 440

(2) INFORMATION FOR SEQ ID NO: 5:

(i) SEQUENCE CHARACTERISTICS:
(A) LENGTH: 444 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION:1..444

(xi) SEQUENCE DESCRIPTION: SEQ ID NO: 5:

Met Gly Lys Gly Gly Asn Gln Gly Glu Gly Ala Ala Glu Arg Glu Val
1               5                   10                  15

Ser Val Pro Thr Phe Ser Trp Glu Glu Ile Gln Lys His Asn Leu Arg
            20                  25                  30

Thr Asp Arg Trp Leu Val Ile Asp Arg Lys Val Tyr Asn Ile Thr Lys
        35                  40                  45

Trp Ser Ile Gln His Pro Gly Gly Gln Arg Val Ile Gly His Tyr Ala
    50                  55                  60

Gly Glu Asp Ala Thr Asp Ala Phe Arg Ala Phe His Pro Asp Leu Glu
65                  70                  75                  80

Phe Val Gly Lys Phe Leu Lys Pro Leu Leu Ile Gly Glu Leu Ala Pro
                85                  90                  95

Glu Glu Pro Ser Gln Asp His Gly Lys Asn Ser Lys Ile Thr Glu Asp
            100                 105                 110

Phe Arg Ala Leu Arg Lys Thr Ala Glu Asp Met Asn Leu Phe Lys Thr
        115                 120                 125

Asn His Val Phe Phe Leu Leu Leu Leu Ala His Ile Ile Ala Leu Glu
    130                 135                 140

Ser Ile Ala Trp Phe Thr Val Phe Tyr Phe Gly Asn Gly Trp Ile Pro
145                 150                 155                 160

Thr Leu Ile Thr Ala Phe Val Leu Ala Thr Ser Gln Ala Gln Ala Gly
                165                 170                 175

Trp Leu Gln His Asp Tyr Gly His Leu Ser Val Tyr Arg Lys Pro Lys
            180                 185                 190

Trp Asn His Leu Val His Lys Phe Val Ile Gly His Leu Lys Gly Ala
        195                 200                 205

Ser Ala Asn Trp Trp Asn His Arg His Phe Gln His His Ala Lys Pro
    210                 215                 220

Asn Ile Phe His Lys Asp Pro Asp Val Asn Met Leu His Val Phe Val
225                 230                 235                 240

Leu Gly Glu Trp Gln Pro Ile Glu Tyr Gly Lys Lys Lys Leu Lys Tyr
                245                 250                 255

Leu Pro Tyr Asn His Gln His Glu Tyr Phe Phe Leu Ile Gly Pro Pro
            260                 265                 270

Leu Leu Ile Pro Met Tyr Phe Gln Tyr Gln Ile Ile Met Thr Met Ile
        275                 280                 285

Val His Lys Asn Trp Val Asp Leu Ala Trp Ala Val Ser Tyr Tyr Ile
    290                 295                 300

Arg Phe Phe Ile Thr Tyr Ile Pro Phe Tyr Gly Ile Leu Gly Ala Leu
305                 310                 315                 320

Leu Phe Leu Asn Phe Ile Arg Phe Leu Glu Ser His Trp Phe Val Trp
                325                 330                 335

Val Thr Gln Met Asn His Ile Val Met Glu Ile Asp Gln Glu Ala Tyr
            340                 345                 350

Arg Asp Trp Phe Ser Ser Gln Leu Thr Ala Thr Cys Asn Val Glu Gln
        355                 360                 365

Ser Phe Phe Asn Asp Trp Phe Ser Gly His Leu Asn Phe Gln Ile Glu
    370                 375                 380

His His Leu Phe Pro Thr Met Pro Arg His Asn Leu His Lys Ile Ala
385                 390                 395                 400

Pro Leu Val Lys Ser Leu Cys Ala Lys His Gly Ile Glu Tyr Gln Glu
                405                 410                 415

Lys Pro Leu Leu Arg Ala Leu Leu Asp Ile Ile Arg Ser Leu Lys Lys
            420                 425                 430

Ser Gly Lys Leu Trp Leu Asp Ala Tyr Leu His Lys
        435                 440

(2) INFORMATION FOR SEQ ID NO: 6:

(i) SEQUENCE CHARACTERISTICS:
(A) LENGTH: 445 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION:1..445

(xi) SEQUENCE DESCRIPTION: SEQ ID NO: 6:

Met Gly Gly Val Gly Glu Pro Gly Pro Arg Glu Gly Pro Ala Gln Pro
1               5                   10                  15

Gly Ala Pro Leu Pro Thr Phe Cys Trp Glu Gln Ile Arg Ala His Asp
            20                  25                  30

Gln Pro Gly Asp Lys Trp Leu Val Ile Glu Arg Arg Val Tyr Asp Ile
        35                  40                  45

Ser Arg Trp Ala Gln Arg His Pro Gly Gly Ser Arg Leu Ile Gly His
    50                  55                  60

His Gly Ala Glu Asp Ala Thr Asp Ala Phe Arg Ala Phe His Gln Asp
65                  70                  75                  80

Leu Asn Phe Val Arg Lys Phe Leu Gln Pro Leu Leu Ile Gly Glu Leu
                85                  90                  95

Ala Pro Glu Glu Pro Ser Gln Asp Gly Pro Leu Asn Ala Gln Leu Val
            100                 105                 110

Glu Asp Phe Arg Ala Leu His Gln Ala Ala Glu Asp Met Lys Leu Phe
        115                 120                 125

Asp Ala Ser Pro Thr Phe Phe Ala Phe Leu Leu Gly His Ile Leu Ala
    130                 135                 140

Met Glu Val Leu Ala Trp Leu Leu Ile Tyr Leu Leu Gly Pro Gly Trp
145                 150                 155                 160

Val Pro Ser Ala Leu Ala Ala Phe Ile Leu Ala Ile Ser Gln Ala Gln
                165                 170                 175

Ser Trp Cys Leu Gln His Asp Leu Gly His Ala Ser Ile Phe Lys Lys
            180                 185                 190

Ser Trp Trp Asn His Val Ala Gln Lys Phe Val Met Gly Gln Leu Lys
        195                 200                 205

Gly Phe Ser Ala His Trp Trp Asn Phe Arg His Phe Gln His His Ala
    210                 215                 220

Lys Pro Asn Ile Phe His Lys Asp Pro Asp Val Thr Val Ala Pro Val
225                 230                 235                 240

Phe Leu Leu Gly Glu Ser Ser Val Glu Tyr Gly Lys Lys Lys Arg Arg
                245                 250                 255

Tyr Leu Pro Tyr Asn Gln Gln His Leu Tyr Phe Phe Leu Ile Gly Pro
            260                 265                 270

Pro Leu Leu Thr Leu Val Asn Phe Glu Val Glu Asn Leu Ala Tyr Met
        275                 280                 285

Leu Val Cys Met Gln Trp Ala Asp Leu Leu Trp Ala Ala Ser Phe Tyr
    290                 295                 300

Ala Arg Phe Phe Leu Ser Tyr Leu Pro Phe Tyr Gly Val Pro Gly Val
305                 310                 315                 320

Leu Leu Phe Phe Val Ala Val Arg Val Leu Glu Ser His Trp Phe Val
                325                 330                 335

Trp Ile Thr Gln Met Asn His Ile Pro Lys Glu Ile Gly His Glu Lys
            340                 345                 350

His Arg Asp Trp Val Ser Ser Gln Leu Ala Ala Thr Cys Asn Val Glu
        355                 360                 365

Pro Ser Leu Phe Thr Asn Trp Phe Ser Gly His Leu Asn Phe Gln Ile
    370                 375                 380

Glu His His Leu Phe Pro Arg Met Pro Arg His Asn Tyr Ser Arg Val
385                 390                 395                 400

Ala Pro Leu Val Lys Ser Leu Cys Ala Lys His Gly Leu Ser Tyr Glu
                405                 410                 415

Val Lys Pro Phe Leu Thr Ala Leu Val Asp Ile Val Arg Ser Leu Lys
            420                 425                 430

Lys Ser Gly Asp Ile Trp Leu Asp Ala Tyr Leu His Gln
        435                 440                 445

(2) INFORMATION FOR SEQ ID NO: 7:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..18

(xi) SEQUENCE DESCRIPTION: SEQ ID NO: 7:

TCGTCCCCGA CAGTCCGC 18

(2) INFORMATION FOR SEQ ID NO: 8:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 8:

TAAAACACTA ACCGGTGGAC 20

(2) INFORMATION FOR SEQ ID NO: 9:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..22

(xi) SEQUENCE DESCRIPTION: SEQ ID NO: 9:

ATCGTCTACC AAGGTGAAAC TC 22

(2) INFORMATION FOR SEQ ID NO: 10:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 10:

CCTTGTACCT GACCCAAAGG 20

(2) INFORMATION FOR SEQ ID NO: 11:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..21

(xi) SEQUENCE DESCRIPTION: SEQ ID NO: 11:

TGATACTTCT TTTCCGGGTC C 21

(2) INFORMATION FOR SEQ ID NO: 12:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..19

(xi) SEQUENCE DESCRIPTION: SEQ ID NO: 12:

TCCGTCTCCC CTCGAACTC 19

(2) INFORMATION FOR SEQ ID NO: 13:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..19

(xi) SEQUENCE DESCRIPTION: SEQ ID NO: 13:

CGCGTCCAAT ACGAAGACT 19

(2) INFORMATION FOR SEQ ID NO: 14:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 14:

GGACGACTAA CCACTTGACC 20

(2) INFORMATION FOR SEQ ID NO: 15:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 15:

TCAAGTGGTT AGTCGTCCCC 20

(2) INFORMATION FOR SEQ ID NO: 16:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 16:

CGACTTTATG GACGGGATGT 20

(2) INFORMATION FOR SEQ ID NO: 17:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..19

(xi) SEQUENCE DESCRIPTION: SEQ ID NO: 17:

AACGGCATGA GCTACCCGA 19

(2) INFORMATION FOR SEQ ID NO: 18:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 18:

CTACCCGAAA ACAAGACTCC 20

(2) INFORMATION FOR SEQ ID NO: 19:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..18

(xi) SEQUENCE DESCRIPTION: SEQ ID NO: 19:

CGGCTCCAGG CTCGTTCT 18

(2) INFORMATION FOR SEQ ID NO: 20:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 20:

GTGACCAAGC ACACCTAGTG 20

(2) INFORMATION FOR SEQ ID NO: 21:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..19

(xi) SEQUENCE DESCRIPTION: SEQ ID NO: 21:

GTAGGACCGA CCCAAGAAG 19

(2) INFORMATION FOR SEQ ID NO: 22:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 22:

CTCTTAACCC CCTTTCGACA 20

SEQUENCE LISTING

<110> MultiGene Biotech GmbH

<120> cDNA molecules of the members of gene family encoding
      human fatty acid desaturases and their use in diagnosis
      and therapy

<130> MultiGene Anm. 99/002 EP

<140> 99104664.0-2105
<141> 1999-03-09

<160> 22

<170> PatentIn Ver. 2.1

<210> 1
<211> 4205
<212> DNA
<213> Unknown Organism

<220>
<223> Description of Unknown Organism:

      unknown

<400> 1
cactcctgga gcccgcggac cccgagcacg cgcctgacag cccctgctgg cccggcgcgc 60
ggcgtcgcca ggccagctat ggcccccgac ccggtggccg ccgagaccgc ggctcaggga 120
cctaccccgc gctacttcac ctgggacgag gtggcccagc gctcagggtg cgaggagcgg 180
tggctagtga tcgaccgtaa ggtgtacaac atcagcgagt tcacccgccg gcatccaggg 240
ggctcccggg tcatcagcca ctacgccggg caggatgcca cggatccctt tgtggccttc 300
cacatcaaca agggccttgt gaagaagtat atgaactctc tcctgattgg agaactgtct 360
ccagagcagc ccagctttga gcccaccaag aataaagagc tgacagatga gttccgggag 420
ctgcgggcca cagtggagcg gatggggctc atgaaggcca accatgtctt cttcctgctg 480
tacctgctgc acatcttgct gctggatggt gcagcctggc tcaccctttg ggtctttggg 540
acgtcctttt tgcccttcct cctctgtgcg gtgctgctca gtgcagttca ggcccaggct 600
ggctggctgc agcatgactt tgggcacctg tcggtcttca gcacctcaaa gtggaaccat 660
ctgctacatc attttgtgat tggccacctg aagggggccc ccgccagttg gtggaaccac 720
atgcacttcc agcaccatgc caagcccaac tgcttccgca aagacccaga catcaacatg 780
catcccttct tctttgcctt ggggaagatc ctctctgtgg agcttgggaa acagaagaaa 840

aaatatatgc cgtacaacca ccagcacaaa tacttcttcc taattgggcc cccagccttg 900
ctgcctctct acttccagtg gtatattttc tattttgtta tccagcgaaa gaagtgggtg 960
gacttggcct ggatgattac cttctacgtc cgcttcttcc tcacttatgt gccactattg 1020
gggctgaaag ccttcctggg ccttttcttc atagtcaggt tcctggaaag caactggttt 1080
gtgtgggtga cacagatgaa ccatattccc atgcacattg atcatgaccg gaacatggac 1140
tgggtttcca cccagctcca ggccacatgc aatgtccaca agtctgcctt caatgactgg 1200
ttcagtggac acctcaactt ccagattgag caccatcttt ttcccacgat gcctcgacac 1260
aattaccaca aagtggctcc cctggtgcag tccttgtgtg ccaagcatgg catagagtac 1320
cagtccaagc ccctgctgtc agccttcgcc gacatcatcc actcactaaa ggagtcaggg 1380
cagctctggc tagatgccta tcttcaccaa taacaacagc caccctgccc agtctggaag 1440
aagaggagga agactctgga gccaaggcag aggggagctt gagggacaat gccactatag 1500
tttaatactc agagggggtt gggtttgggg acataaagcc tctgactcaa actcctccct 1560
tttatcttct agccacagtt ctaagaccca aagtgggggg tggacacaga agtccctagg 1620
agggaaggag ctgttggggc aggggtgtaa attatttcct ttttctagtt tggcacatgc 1680
aggtagttgg tgaacagaga gaaccaggag ggtaacagaa gaggagggac ctactgaacc 1740
cagagtcagg aagagattta acactaaaat tccactcatg ccgggcgtgg tgcacgcgcc 1800
tgtaatccca gctacccagg aggctgaggc aggagaatcg cttgaaccgg ggaggtggag 1860
gttgcagtga gctgagatca cgccattgta ctccagcctg ggcgacagag caagactcca 1920
tttcaaaaaa aaaaaaaaaa aaaaaaatcc actcatataa aaggtgagct cagctcactg 1980
gtccatttct cagtggcttc tccatcctca tttgcaaacc tcagagggat aaggcagttg 2040
aacctgatga gcaagaatta taacagcaag gaaacattaa tgcttagaat tctgagatcc 2100
agcacaactc agtctgtggg agctcagctc gctgcccagg gataggtatg acctatgtct 2160
gccttaggct gctgggagat gccattctcc agtttcagaa gcaggcaggg caaaggtcaa 2220
gactgtggta ttggggtctt ttggctctga aggatcctgg aaccactgat tttggtttat 2280
tccctccagg gtctaaagag aacaagaggt gctagctctt accaaaacag atggtagaga 2340
gagttgctgg ctatttaaaa agctctttca tcttttaatt cacctcttct tttcacctct 2400
ttaaccactc ctcaggaaca gaacacttct aggactgggg gtcttttagc tccataagca 2460
agtgagcaga tgggacaagt tagtcttttc tccctagaaa caaaggggat gcccagtggt 2520
ttccctttgc ttcccaacct aaaatttcaa gtttaataaa atagcaatta gcagaagtga 2580
ccaaattggg agataattat cagtcatgag gaaagacaca gatttcggtc ataaagaatg 2640
taagggctat aagtagaaac tttctataac ctaaatgatg ttatagaatt atttttgagc 2700
aggagcagaa agattaaata tgatcacttc atacttctaa atcagaaata ggaagattaa 2760
aaccacagaa cagtttgtga tttctattgc tggtagctag gtatcttact ctgtccactc 2820
ttgttcaagt atctaactct tctggaaacc aaataggctt tagaagagat tatcctatat 2880
tcctatcagt ataatactaa aatgtaactt tttaatcatc tggtttttaa aagataaaca 2940
gtttagccca tctctccaga gagcaaacat aggaatatga ctcaggagcc tcctagggct 3000
tatcatcagc cctcacaccc gcttccccct ccaacccaca gcctttgctt ccaggtggca 3060
ggattactac tttgcctctt cagcagcatc tactctaggc atattgatca ttttagacac 3120
tgggagaaga gaacctcaaa ctaggaggaa aagacagagc ctccacttag ttttgggagg 3180
ggatggcaga cagtcaagga gatgagcgtc ctaaggcatg ttgggatagg gtcagatgca 3240
ccacccatgg agaggtttgt caacacaaag acatggaagg ttagaggttt gtcaacaaaa 3300
agacatggaa ggttaggttt gtcaacacaa agacatggaa gattagaggt ttgtcaacac 3360

aaagatacag gaagaatggg ctgcagaaga tttagatgtt ttccatttgg gcacatttta 3420
cttagctgga gaactaggtt taaaacagcc tgggtaggaa aattagaagc aagctggatg 3480
cagtggctca tgcctgtaat cccaacactt ttgggaggtc caggcaggag gatcacttgg 3540
gcccaggagg tcaagcctgc agcgagctga gatcacacca ctgcactcca gcctggggtg 3600
atagaacaag accctgtctc aaaaaaaaaa aaaaacaaca aaaacttaga attgaggagt 3660
tgtacctcca ttggcttcct cactccaaaa taggtgctga tccttcctat tcctattctt 3720
tgccaccttt tgggtgtggt gtcaccagcc tgtttagcca agtagctttg ggcataggct 3780
gcccaatctg agcaaacacc agtgaggctc tattgagcaa gaccaagtcc tcaaagcacc 3840
tgaaccactg tggccttctc agcctacagc agtgtggtct cttacatggc cacaaaggga 3900
cacacagtga caaaaggctc ggaatgttac aatggtaaaa tgagtgatct caaatccact 3960
gacagatata aaataggctt agagaggaaa agctgcctct ggtcaagtag atcatggcag 4020
catgaattcc aactcacttt tttacgaact ccaacttcta tgtttatctt tgttactttc 4080
acttttttac aacctgncag aggcattttt taaatcaggc ccaatatcag tattcttttt 4140
gtgtgtgcca attttgttat cacatcccta tgaagttgaa aaataaagtt aattttgacc 4200
aaaag 4205

<210> 2
<211> 4089
<212> DNA
<213> Unknown Organism

<220>
<223> Description of Unknown Organism:

unknown

<400> 2
cgtcacagtc ggcaggcagc atggggaagg gagggaacca gggcgagggg gccgccgagc 60
gcgaggtgtc ggtgcccacc ttcagctggg aggagattca gaagcataac ctgcgcaccg 120
acaggtggct ggtcattgac cgcaaggttt acaacatcac caaatggtcc atccagcacc 180
cggggggcca gcgggtcatc gggcactacg ctggagaaga tgcaacggat gccttccgcg 240
ccttccaccc tgacctggaa ttcgtgggca agttcttgaa acccctgctg attggtgaac 300
tggccccgga ggagcccagc caggaccacg gcaagaactc aaagatcact gaggacttcc 360
gggccctgag gaagacggct gaggacatga acctgttcaa gaccaaccac gtgttcttcc 420
tcctcctcct ggcccacatc atcgccctgg agagcattgc atggttcact gtcttttact 480
ttggcaatgg ctggattcct accctcatca cggcctttgt ccttgctacc tctcaggccc 540
aagctggatg gctgcaacat gattatggcc acctgtctgt ctacagaaaa cccaagtgga 600
accaccttgt ccacaaattc gtcattggcc acttaaaggg tgcctctgcc aactggtgga 660
atcatcgcca cttccagcac cacgccaagc ctaacatctt ccacaaggat cccgatgtga 720
acatgctgca cgtgtttgtt ctgggcgaat ggcagcccat cgagtacggc aagaagaagc 780
tgaaatacct gccctacaat caccagcacg aatacttctt cctgattggg ccgccgctgc 840
tcatccccat gtatttccag taccagatca tcatgaccat gatcgtccat aagaactggg 900

tggacctggc ctgggccgtc agctactaca tccggttctt catcacctac atccctttct 960
acggcatcct gggagccctc cttttcctca acttcatcag gttcctggag agccactggt 1020
ttgtgtgggt cacacagatg aatcacatcg tcatggagat tgaccaggag gcctaccgtg 1080
actggttcag tagccagctg acagccacct gcaacgtgga gcagtccttc ttcaacgact 1140
ggttcagtgg acaccttaac ttccagattg agcaccacct cttccccacc atgccccggc 1200
acaacttaca caagatcgcc ccgctggtga agtctctatg tgccaagcat ggcattgaat 1260
accaggagaa gccgctactg agggccctgc tggacatcat caggtccctg aagaagtctg 1320
ggaagctgtg gctggacgcc taccttcaca aatgaagcca cagcccccgg gacaccgtgg 1380
ggaaggggtg caggtggggt gatggccaga ggaatgatgg gcttttgttc tgaggggtgt 1440
ccgagaggct ggtgtatgca ctgctcacgg accccatgtt ggatctttct ccctttctcc 1500
tctccttttt ctcttcacat ctcccccata gcaccctgcc ctcatgggac ctgccctccc 1560
tcagccgtca gccatcagcc atggccctcc cagtgcctcc tagccccttc ttccaaggag 1620
cagagaggtg gccaccgggg gtggctctgt cctacctcca ctctctgccc ctaaagatgg 1680
gaggagacca gcggtccatg ggtctggcct gtgagtctcc ccttgcagcc tggtcactag 1740
gcatcacccc cgctttggtt cttcagatgc tcttggggtt cataggggca ggtcctagtc 1800
gggcagggcc cctgaccctc ccggcctggc ttcactctcc ctgacggctg ccattggtcc 1860
accctttcat agagaggcct gctttgttac aaagctcggg tctccctcct gcagctcggt 1920
taagtacccg aggcctctct taagatgtcc agggccccag gcccgcgggc acagccagcc 1980
caaaccttgg gccctggaag agtcctccac cccatcacta gagtgctctg accctgggct 2040
ttcacgggcc ccattccacc gcctccccaa cttgagcctg tgaccttggg accaaagggg 2100
gagtccctcg tctcttgtga ctcagcagag gcagtggcca cgttcaggga ggggccggct 2160
ggcctggagg ctcagcccac cctccagctt ttcctcaggg tgtcctgagg tccaagattc 2220
tggagcaatc tgacccttct ccaaaggctc tgttatcagc tgggcagtgc cagccaatcc 2280
ctggccattt ggccccaggg gacgtgggcc ctgcaggctg caggagggca ctggagctgg 2340
gaggtctcgt cccagccctc cccatctcgg ggctgctgtg tggacggcgc tgcctcaggc 2400
actctcctgt ctgaacctgc ccttactgtg tttaacctgt tgctccagga tgcattctga 2460
taggaggggg cggcagggct gggccttgtg acaatctgcc tttcaccaca tggccttgcc 2520
tcggtggccc tgactgtcag ggagggccag ggaggcagag cgggagggag tctcaggagg 2580
aggctgccct gaggggctgg ggagggggta cctcatgagg accagggtgg agctgagaag 2640
aggaggaggt gggggctgga ggtgctggta gctgagggga cgggcaagtg agaggggagg 2700
gagggaagtc ctgggaggat cctgagctgc tgttgcagtc taacccacta atcagttctt 2760
agattcaggg gaagggcagg caccaacaac tcagaatggg ggctttcggg gagggcgcct 2820
agtcccccca gctctaagca gccaggaggg acctgcatct aagcatctgg gttgccatgg 2880
caatggcatg ccccccagct actgtatgcc cccgaccccc gcagaggcag aatgaaccca 2940
tagggagctg atcgtaatgt ttatcatgtt acttccccac ccctacattt tttgaaataa 3000
aataaggaat tttattctca cttcctgtgt ttcctgcacg ccaatgccag gccatggtat 3060
tgggtgatag atgaggccct tctagctggg cctgggcacc aggaggggtc cccatgcttg 3120
catctctctg tatcccctcc ctcccctgtg gccatcccac ccgcctctcc ctgctgcctc 3180
tgaaattcat tctggggccc ggaacttggt ggaaatgacc caaaaacatt ggcccatctt 3240
cctcctctca gcagccgacc ccagcccaat tctaaaacag ggctgagagc cacctctcag 3300
cagctgaccc ctacccaagg agggtggcat ggaggggctt gcagagactc ttcctaacat 3360
cctccccccc cagctgtctc cccaagtgca atctgccctc ccatccctgg gccagccagc 3420

ttccacagag cgccaggcca aacagaattc ctggcctcct tggaaggggc tggagaaggc 3480
cgggagcagt ggctcacgcc tgtaatccca gcactttggg aggctgaggc gggcagatca 3540
caaagtcaag agattgagac catcctggcc aacatggtga aaccccgtct ctactaaaaa 3600
tacaaaaatt aggccgggtg cggtggctca cgcctgtaat cccagcactt tgggaggccg 3660
aggcgggcag atcacgaggt caggagatca agaccatcct ggctaacacg gtgaaacccc 3720
gtctctacta aaaatacaaa aaattagctg ggcgaggtgg cgggtgcctg tagtcccagc 3780
tacgtgggag gctgaggcaa gagaatggcg tgaaccccgg cggggcagag cctgcagaga 3840
gctgagatca caccactgta ctccagcctg ggcgacagcg agactccgtc tcaaaaaaaa 3900
aaaaaaaaaa aattagctgg gcatggtggt gcgtgcctgc agtcccagct actcaggagg 3960
ctgagacggg agaatcgctt gaacctggga ggcagaggtt gcagtgagcc aagatcgctc 4020
actccagcct agcgacagag tgagactcca tctcaaataa ataaataaat taattaatta 4080
aattaaatt 4089

<210> 3
<211> 1757
<212> DNA
<213> Unknown Organism

<220>
<223> Description of Unknown Organism:

unknown

<400> 3
   ggccgcggcg gcagggcggg gccggagcag cgggcggcgg cggaggcggc gcccgggagc 60
   gctcttcgct tccctcgggg tcttgctcgg acctcggcca ccgcctggga tccccaggac 120
   tcgtgcgtgc agcatggcgg gcgtcgggga gccgggaccg cgggagggac ccgcgcagcc 180
   gggggcaccg ctgcccacct tctgctggga gcagatccgc gcgcacgacc agcccggcga 240
   caagtggctg gtcatcgagc gccgcgtcta cgacatcagc cgctgggcac agcggcaccc 300
   agggggcagc cgcctcatcg gccaccacgg cgctgaggac gccacggatg ccttccgtgc 360
   cttccatcaa gatctcaatt ttgtgcgcaa gttcctacag cccctgttga ttggagagct 420
   ggctccggaa gaacccagcc aggatggacc cctgaatgcg cagctggtcg aggacttccg 480
   agccctgcac caggcagccg aggacatgaa gctgtttgat gccagtccca ccttctttgc 540
   tttcctactg ggccacatcc tggccatgga ggtgctggcc tggctcctta tctacctcct 600
   gggtcctggc tgggtgccca gtgccctggc cgccttcatc ctggccatct ctcaggctca 660
   gtcctggtgt ctgcagcatg acctgggcca tgcctccatc ttcaagaagt cctggtggaa 720
   ccacgtggcc cagaagttcg tgatggggca gctaaagggc ttctccgccc actggtggaa 780
   cttccgccac ttccagcacc acgccaagcc caacatcttc cacaaagacc cagacgtgac 840
   ggtggcgccc gtcttcctcc tgggggagtc atccgtcgag tatggcaaga agaaacgcag 900
   atacctaccc tacaaccagc agcacctgta cttcttcctg atcggcccgc cgctgctcac 960
   cctggtgaac tttgaagtgg aaaatctggc gtacatgctg gtgtgcatgc agtgggcgga 1020
   tttgctctgg gccgccagct tctatgcccg cttcttctta tcctacctcc ccttctacgg 1080
   cgtccctggg gtgctgctct tctttgttgc tgtcagggtc ctggaaagcc actggttcgt 1140
   gtggatcaca cagatgaacc acatccccaa ggagatcggc cacgagaagc accgggactg 1200
   ggtcagctct cagctggcag ccacctgcaa cgtggagccc tcacttttca ccaactggtt 1260
   cagcgggcac ctcaacttcc agatcgagca ccacctcttc cccaggatgc cgagacacaa 1320
   ctacagccgg gtggccccgc tggtcaagtc gctgtgtgcc aagcacggcc tcagctacga 1380
   agtgaagccc ttcctcaccg cgctggtgga catcgtcagg tccctgaaga agtctggtga 1440
   catctggctg gacgcctacc tccatcagtg aaggcaacac ccaggcgggc agagaagggc 1500
   tcagggcacc agcaaccaag ccagccccgg cgggatcgat acccccaccc ctccactggc 1560
   cagcctgggg gtgccctgcc tgccctcctg gtactgttgt cttcccctcg gccccctcac 1620
   atgtgtattc agcagcccta tggccttggc tctgggcctg atgggacagg ggtagaggga 1680
   aggtgagcat agcacatttt cctagagcga gaattggggg aaagctgtta tttttatatt 1740
   aaaatacatt cagatgt 1757

<210> 4
<211> 444
<212> PRT
<213> Unknown Organism

<220>
<223> Description of Unknown Organism:

      unknown

<400> 4
Met Ala Pro Asp Pro Val Ala Ala Glu Thr Ala Ala Gln Gly Pro Thr
1               5                   10                  15

Pro Arg Tyr Phe Thr Trp Asp Glu Val Ala Gln Arg Ser Gly Cys Glu
            20                  25                  30

Glu Arg Trp Leu Val Ile Asp Arg Lys Val Tyr Asn Ile Ser Glu Phe
        35                  40                  45

Thr Arg Arg His Pro Gly Gly Ser Arg Val Ile Ser His Tyr Ala Gly
    50                  55                  60

Gln Asp Ala Thr Asp Pro Phe Val Ala Phe His Ile Asn Lys Gly Leu
65                  70                  75                  80

Val Lys Lys Tyr Met Asn Ser Leu Leu Ile Gly Glu Leu Ser Pro Glu
                85                  90                  95

Gln Pro Ser Phe Glu Pro Thr Lys Asn Lys Glu Leu Thr Asp Glu Phe
            100                 105                 110

Arg Glu Leu Arg Ala Thr Val Glu Arg Met Gly Leu Met Lys Ala Asn
        115                 120                 125

His Val Phe Phe Leu Leu Tyr Leu Leu His Ile Leu Leu Leu Asp Gly
    130                 135                 140

Ala Ala Trp Leu Thr Leu Trp Val Phe Gly Thr Ser Phe Leu Pro Phe
145                 150                 155                 160

Leu Leu Cys Ala Val Leu Leu Ser Ala Val Gln Ala Gln Ala Gly Trp
                165                 170                 175

Leu Gln His Asp Phe Gly His Leu Ser Val Phe Ser Thr Ser Lys Trp
            180                 185                 190

Asn His Leu Leu His His Phe Val Ile Gly His Leu Lys Gly Ala Pro
        195                 200                 205

Ala Ser Trp Trp Asn His Met His Phe Gln His His Ala Lys Pro Asn
    210                 215                 220

Cys Phe Arg Lys Asp Pro Asp Ile Asn Met His Pro Phe Phe Phe Ala
225                 230                 235                 240

Leu Gly Lys Ile Leu Ser Val Glu Leu Gly Lys Gln Lys Lys Lys Tyr
                245                 250                 255

Met Pro Tyr Asn His Gln His Lys Tyr Phe Phe Leu Ile Gly Pro Pro
            260                 265                 270

Ala Leu Leu Pro Leu Tyr Phe Gln Trp Tyr Ile Phe Tyr Phe Val Ile
        275                 280                 285

Gln Arg Lys Lys Trp Val Asp Leu Ala Trp Met Ile Thr Phe Tyr Val
    290                 295                 300

Arg Phe Phe Leu Thr Tyr Val Pro Leu Leu Gly Leu Lys Ala Phe Leu
305                 310                 315                 320

Gly Leu Phe Phe Ile Val Arg Phe Leu Glu Ser Asn Trp Phe Val Trp
                325                 330                 335

Val Thr Gln Met Asn His Ile Pro Met His Ile Asp His Asp Arg Asn
            340                 345                 350

Met Asp Trp Val Ser Thr Gln Leu Gln Ala Thr Cys Asn Val His Lys
        355                 360                 365

Ser Ala Phe Asn Asp Trp Phe Ser Gly His Leu Asn Phe Gln Ile Glu
    370                 375                 380

His His Leu Phe Pro Thr Met Pro Arg His Asn Tyr His Lys Val Ala
385                 390                 395                 400

Pro Leu Val Gln Ser Leu Cys Ala Lys His Gly Ile Glu Tyr Gln Ser
                405                 410                 415

Lys Pro Leu Leu Ser Ala Phe Ala Asp Ile Ile His Ser Leu Lys Glu
            420                 425                 430

Ser Gly Gln Leu Trp Leu Asp Ala Tyr Leu His Gln
        435                 440

<210> 5
<211> 444
<212> PRT
<213> Unknown Organism

<220>
<223> Description of Unknown Organism:

unknown

<400> 5
Met Gly Lys Gly Gly Asn Gln Gly Glu Gly Ala Ala Glu Arg Glu Val
1               5                   10                  15

Ser Val Pro Thr Phe Ser Trp Glu Glu Ile Gln Lys His Asn Leu Arg
            20                  25                  30

Thr Asp Arg Trp Leu Val Ile Asp Arg Lys Val Tyr Asn Ile Thr Lys
        35                  40                  45

Trp Ser Ile Gln His Pro Gly Gly Gln Arg Val Ile Gly His Tyr Ala
    50                  55                  60

Gly Glu Asp Ala Thr Asp Ala Phe Arg Ala Phe His Pro Asp Leu Glu
65                  70                  75                  80

Phe Val Gly Lys Phe Leu Lys Pro Leu Leu Ile Gly Glu Leu Ala Pro
                85                  90                  95

Glu Glu Pro Ser Gln Asp His Gly Lys Asn Ser Lys Ile Thr Glu Asp
            100                 105                 110

Phe Arg Ala Leu Arg Lys Thr Ala Glu Asp Met Asn Leu Phe Lys Thr
        115                 120                 125

Asn His Val Phe Phe Leu Leu Leu Leu Ala His Ile Ile Ala Leu Glu
    130                 135                 140

Ser Ile Ala Trp Phe Thr Val Phe Tyr Phe Gly Asn Gly Trp Ile Pro
145                 150                 155                 160

Thr Leu Ile Thr Ala Phe Val Leu Ala Thr Ser Gln Ala Gln Ala Gly
                165                 170                 175

Trp Leu Gln His Asp Tyr Gly His Leu Ser Val Tyr Arg Lys Pro Lys
            180                 185                 190

Trp Asn His Leu Val His Lys Phe Val Ile Gly His Leu Lys Gly Ala
        195                 200                 205

Ser Ala Asn Trp Trp Asn His Arg His Phe Gln His His Ala Lys Pro
    210                 215                 220

Asn Ile Phe His Lys Asp Pro Asp Val Asn Met Leu His Val Phe Val
225                 230                 235                 240

Leu Gly Glu Trp Gln Pro Ile Glu Tyr Gly Lys Lys Lys Leu Lys Tyr
                245                 250                 255

Leu Pro Tyr Asn His Gln His Glu Tyr Phe Phe Leu Ile Gly Pro Pro
            260                 265                 270

Leu Leu Ile Pro Met Tyr Phe Gln Tyr Gln Ile Ile Met Thr Met Ile
        275                 280                 285

Val His Lys Asn Trp Val Asp Leu Ala Trp Ala Val Ser Tyr Tyr Ile
    290                 295                 300

Arg Phe Phe Ile Thr Tyr Ile Pro Phe Tyr Gly Ile Leu Gly Ala Leu
305                 310                 315                 320

Leu Phe Leu Asn Phe Ile Arg Phe Leu Glu Ser His Trp Phe Val Trp
                325                 330                 335

Val Thr Gln Met Asn His Ile Val Met Glu Ile Asp Gln Glu Ala Tyr
            340                 345                 350

Arg Asp Trp Phe Ser Ser Gln Leu Thr Ala Thr Cys Asn Val Glu Gln
        355                 360                 365

Ser Phe Phe Asn Asp Trp Phe Ser Gly His Leu Asn Phe Gln Ile Glu
    370                 375                 380

His His Leu Phe Pro Thr Met Pro Arg His Asn Leu His Lys Ile Ala
385                 390                 395                 400

Pro Leu Val Lys Ser Leu Cys Ala Lys His Gly Ile Glu Tyr Gln Glu
                405                 410                 415

Lys Pro Leu Leu Arg Ala Leu Leu Asp Ile Ile Arg Ser Leu Lys Lys
            420                 425                 430

Ser Gly Lys Leu Trp Leu Asp Ala Tyr Leu His Lys
        435                 440

<210> 6

<211> 445

<212> PRT

<213> Unknown Organism

<220>

<223> Description of Unknown Organism:

      unknown

<400> 6

Met Gly Gly Val Gly Glu Pro Gly Pro Arg Glu Gly Pro Ala Gln Pro
  1               5                  10                  15

Gly Ala Pro Leu Pro Thr Phe Cys Trp Glu Gln Ile Arg Ala His Asp
             20                  25                  30

Gln Pro Gly Asp Lys Trp Leu Val Ile Glu Arg Arg Val Tyr Asp Ile
         35                  40                  45

Ser Arg Trp Ala Gln Arg His Pro Gly Gly Ser Arg Leu Ile Gly His
     50                  55                  60

His Gly Ala Glu Asp Ala Thr Asp Ala Phe Arg Ala Phe His Gln Asp
 65                  70                  75                  80

Leu Asn Phe Val Arg Lys Phe Leu Gln Pro Leu Leu Ile Gly Glu Leu
                 85                  90                  95

Ala Pro Glu Glu Pro Ser Gln Asp Gly Pro Leu Asn Ala Gln Leu Val
            100                 105                 110

Glu Asp Phe Arg Ala Leu His Gln Ala Ala Glu Asp Met Lys Leu Phe
        115                 120                 125

Asp Ala Ser Pro Thr Phe Phe Ala Phe Leu Leu Gly His Ile Leu Ala
    130                 135                 140

Met Glu Val Leu Ala Trp Leu Leu Ile Tyr Leu Leu Gly Pro Gly Trp
145                 150                 155                 160

Val Pro Ser Ala Leu Ala Ala Phe Ile Leu Ala Ile Ser Gln Ala Gln
                165                 170                 175

Ser Trp Cys Leu Gln His Asp Leu Gly His Ala Ser Ile Phe Lys Lys
            180                 185                 190

Ser Trp Trp Asn His Val Ala Gln Lys Phe Val Met Gly Gln Leu Lys
        195                 200                 205

Gly Phe Ser Ala His Trp Trp Asn Phe Arg His Phe Gln His His Ala
    210                 215                 220

Lys Pro Asn Ile Phe His Lys Asp Pro Asp Val Thr Val Ala Pro Val
225                 230                 235                 240

Phe Leu Leu Gly Glu Ser Ser Val Glu Tyr Gly Lys Lys Lys Arg Arg
                245                 250                 255

Tyr Leu Pro Tyr Asn Gln Gln His Leu Tyr Phe Phe Leu Ile Gly Pro
            260                 265                 270

Pro Leu Leu Thr Leu Val Asn Phe Glu Val Glu Asn Leu Ala Tyr Met
        275                 280                 285

Leu Val Cys Met Gln Trp Ala Asp Leu Leu Trp Ala Ala Ser Phe Tyr
    290                 295                 300

Ala Arg Phe Phe Leu Ser Tyr Leu Pro Phe Tyr Gly Val Pro Gly Val
305                 310                 315                 320

Leu Leu Phe Phe Val Ala Val Arg Val Leu Glu Ser His Trp Phe Val
                325                 330                 335

Trp Ile Thr Gln Met Asn His Ile Pro Lys Glu Ile Gly His Glu Lys
            340                 345                 350

His Arg Asp Trp Val Ser Ser Gln Leu Ala Ala Thr Cys Asn Val Glu
        355                 360                 365

Pro Ser Leu Phe Thr Asn Trp Phe Ser Gly His Leu Asn Phe Gln Ile
    370                 375                 380

Glu His His Leu Phe Pro Arg Met Pro Arg His Asn Tyr Ser Arg Val
385                 390                 395                 400

Ala Pro Leu Val Lys Ser Leu Cys Ala Lys His Gly Leu Ser Tyr Glu
                405                 410                 415

Val Lys Pro Phe Leu Thr Ala Leu Val Asp Ile Val Arg Ser Leu Lys
            420                 425                 430

Lys Ser Gly Asp Ile Trp Leu Asp Ala Tyr Leu His Gln
        435                 440                 445

<210> 7
<211> 18
<212> DNA
<213> Unknown Organism

<220>
<223> Description of Unknown Organism:

      unknown

<400> 7
cgcctgacag cccctgct                                                18

<210> 8
<211> 20
<212> DNA
<213> Unknown Organism

<220>
<223> Description of Unknown Organism:

      unknown

<400> 8
caggtggcca atcacaaaat                                              20

<210> 9
<211> 22
<212> DNA

<213> Unknown Organism

<220>
<223> Description of Unknown Organism:

      unknown

<400> 9
ctcaaagtgg aaccatctgc ta                                              22

<210> 10
<211> 20
<212> DNA
<213> Unknown Organism

<220>
<223> Description of Unknown Organism:

      unknown

<400> 10
ggaaacccag tccatgttcc                                                 20

<210> 11
<211> 21
<212> DNA
<213> Unknown Organism

<220>
<223> Description of Unknown Organism:

      unknown

<400> 11
cctgggcctt ttcttcatag t                                               21

<210> 12
<211> 19
<212> DNA
<213> Unknown Organism

<220>

<223> Description of Unknown Organism:

      unknown

<400> 12
ctcaagctcc cctctgcct                                                    19

<210> 13
<211> 19
<212> DNA
<213> Unknown Organism

<220>
<223> Description of Unknown Organism:

      unknown

<400> 13
tcagaagcat aacctgcgc                                                    19

<210> 14
<211> 20
<212> DNA
<213> Unknown Organism

<220>
<223> Description of Unknown Organism:

      unknown

<400> 14
ccagttcacc aatcagcagg                                                   20

<210> 15
<211> 20
<212> DNA
<213> Unknown Organism

<220>
<223> Description of Unknown Organism:

      unknown

<400> 15
cccctgctga ttggtgaact                                                    20

<210> 16
<211> 20
<212> DNA
<213> Unknown Organism

<220>
<223> Description of Unknown Organism:

      unknown

<400> 16
tgtagggcag gtatttcagc                                                    20

<210> 17
<211> 19
<212> DNA
<213> Unknown Organism

<220>
<223> Description of Unknown Organism:

      unknown

<400> 17
agcccatcga gtacggcaa                                                     19

<210> 18
<211> 20
<212> DNA
<213> Unknown Organism

<220>
<223> Description of Unknown Organism:

      unknown

<400> 18
cctcagaaca aaagcccatc                                                    20

<210> 19
<211> 18
<212> DNA
<213> Unknown Organism

<220>
<223> Description of Unknown Organism:

      unknown

<400> 19
tcttgctcgg acctcggc                                                    18

<210> 20
<211> 20
<212> DNA
<213> Unknown Organism

<220>
<223> Description of Unknown Organism:

      unknown

<400> 20
gtgatccaca cgaaccagtg                                                  20

<210> 21
<211> 19
<212> DNA
<213> Unknown Organism

<220>
<223> Description of Unknown Organism:

      unknown

<400> 21
gaagaaccca gccaggatg                                                   19

<210> 22

<211> 20

<212> DNA

<213> Unknown Organism

<220>

<223> Description of Unknown Organism:

unknown

<400> 22

acagctttcc cccaattctc 20

## Description

### Field of the invention

The present invention relates to the cloning and sequencing of the cDNA molecules of three members of a gene family encoding three human fatty acid desaturases, fatty acid desaturase-1 (FADS1), fatty acid desaturase-2 (FADS2) and fatty acid desaturase-3 (FADS3). The invention also relates to diagnostic methods of screening for and detection of FADS1, FADS2, FADS3 and gene therapy utilizing recombinant DNA as well as the generation of animal models (knock-in, knock-out, transgenic animals), anti-FADS1, anti-FADS2, anti-FADS3 antibodies and use in screenings for modulating drugs.

### Background of the Invention

Cellular membranes are dynamic structures in which variable amounts of proteins are embedded in a lipid bilayer whose hydrophobic characteristics are largely due to fatty acid moieties of complex lipids (Singer and Nicolson 1972). The 'fluidity' of the membranes are achieved by incorporating unsaturated fatty acyl chains of varying lengths and varying degrees of unsaturation into the lipids (Stubbs and Smith 1984). In animals, some of the unsaturated fatty acids need to be supplied by the diet ('essential polyunsaturated fatty acids') but, in part, can also be synthesized de novo by oxidative desaturation (i.e. formation of double bonds) of saturated fatty acids of plant and animal origin. Polyunsaturated fatty acid formation requires acetyl-CoA dependent chain elongation and desaturation. Most mammalian tissues can modify acyl chains by introducing more than one double bond with the first one generally at the Δ-9 position between carbons C-9 and C-10. Subsequent double bonds may then be inserted at the Δ-4, Δ-5, and Δ-6 positions by individual desaturase activities (Cook 1991).

For the two major precursors of the (n-6) and (n-3) series of polyunsaturated fatty acids, linoleic 18:2(n-6) and alpha-linolenic 18:3(n-3) acids, animals depend entirely on their dietary intake. By alternating sequences of desaturation (involving the subsequent action of Δ4, Δ5- and Δ6-desaturases, respectively) and C2 chain elongation, linoleic and alpha-linolenic acids are utilized to form arachidonic acid, 20:4(n-6), and the (n-3) acyl chains eicosapentaenoic acid, 20:5(n-3), and docosahexaenoic acid, 22:6(n-3), respectively (Cook 1991).

Linoleic and arachidonic acid are the only members of the (n-6) family that accumulate in large quantities in liver and most other animal tissues. The intermediates 18:3(n-6) and 20:3(n-6) are formed from 18:2(n-6) by Δ6-desaturation, chain elongation and Δ5-desaturation (Horrobin 1993). As a component of phospholipids arachidonic acid is abundant in cellular membranes but also serves as the primary precursor of oxygenated derivatives such as prostaglandine E2 which is pro-inflammatory and regulates cell function of the immune system.

The (n-3) acyl chains eicosapentaenoic acid [20:5(n-3)] and docosahexaenoic acid (22:6(n-3)] are most abundant in cerebral cortex, retina, and spermatozoa. Although it is generally assumed that the liver is the major source of 22:6(n-3), it has been shown that docosahexaenoic acid can also be produced by retinal pigment epithelium (Wang and Anderson 1993) as well as brain astrocytes (Moore et al. 1991, Delton-Vandenbrouke et al. 1997). In retinal rod outer segments, phospholipids may contain 40-60% of 22:6(n-3) which can markedly influence membrane fluidity due to the presence of six double bonds.

In recent years there has been increasing interest in the role of polyunsaturated fatty acids in the pathobiology of a number of chronic conditions such as coronary and peripheral vascular disease (Horrobin 1995), acute and chronic inflammatory immune responses (Calder 1998, Fan and Chapkin 1998, Grimble and Tappia 1998), cutaneous abnormalities (Horrobin 1989, Grattan et al. 1990), essential hypertension (Russo et al. 1997, Chi and Gupta 1998), diabetes mellitus (Mori et al. 1997), asthma (Leichsenring et al. 1995, Villani et al. 1998, Hodge et al. 1998) and rheumatoid arthritis (James and Cleland 1997, Ariza-Ariza et al. 1998, Grimble and Tappia 1998). A particular role has been attributed to gamma-linolenic acid [18:3(n-6)] as an anti-cancer polyunsaturated fatty acid. It has been shown that 18:3(n-6) confers anticancer properties by a variety of mechanisms such as (i) up-regulation of E-cadherin, a cell-cell adhesion molecule which acts as a suppressor of metastasis (Jiang et al. 1995), (ii) regulation of desmosome-mediated cell-cell adhesion in human cancer cells (Jiang et al. 1997a), (iii) up-regulation of the metastasis-suppressor gene nm-23 thus contributing to the inhibition of the in vitro invasion of tumor cells (Jiang et al. 1998a), (iv) up-regulation of maspin expression, a mammary serine protease inhibitor, with profound effects on motility of cancer cells (Jiang et al. 1997b) and (v) finally inhibition of cell cycle progression via regulation of phosphorylation and subsequent degradation of cell cycle inhibitors p27kip1 and p57kip2 (Jiang et al. 1998b).

To further understand lipid-related function in human health and disease additional research into fatty acid biosynthesis and metabolism is required. In particular, we need to understand the pharmacological properties, the mechanisms of action and the tissue-specific regulation of composition of the polyunsaturated fatty acids and their metabolites. This will provide additional insight into the role of the polyunsaturated fatty acids in various chronic disease states and will make it feasible to focus pharmacogenomic research on drug design and evaluation with the goal of ameliorating acute health problems associated with impaired lipid function. As a prerequisite, the genes and their gene products involved in the above-mentioned processes need to be identified and characterized.

It is the objective of the present invention to provide cDNA molecules of three novel members of the human membrane fatty acid desaturase gene family, termed FADS1, FADS2 and FADS3. The three genes share a nucleic acid identity of approximately 50-60% and an amino acid identity of about 77% with each other. Similar to other membrane-bound desaturases from mammals, fungi, insects, plants and cyanobacteria FADS1, FADS2 and FADS3 reveal a hydropathy profile typical of membrane-bound desaturases and share three regions of highly conserved primary sequence of the general histidine motif HX₂₍₃₎[XH]H (Shanklin et al. 1994). The histidine residues may act as metal-chelating ligands involved in the binding of oxygen in the reaction center (Shanklin et al. 1995). Together, these features confirm FADS1, FADS2 and FADS3 as novel members of the desaturase family of fatty acyl chain-modifying enzymes.

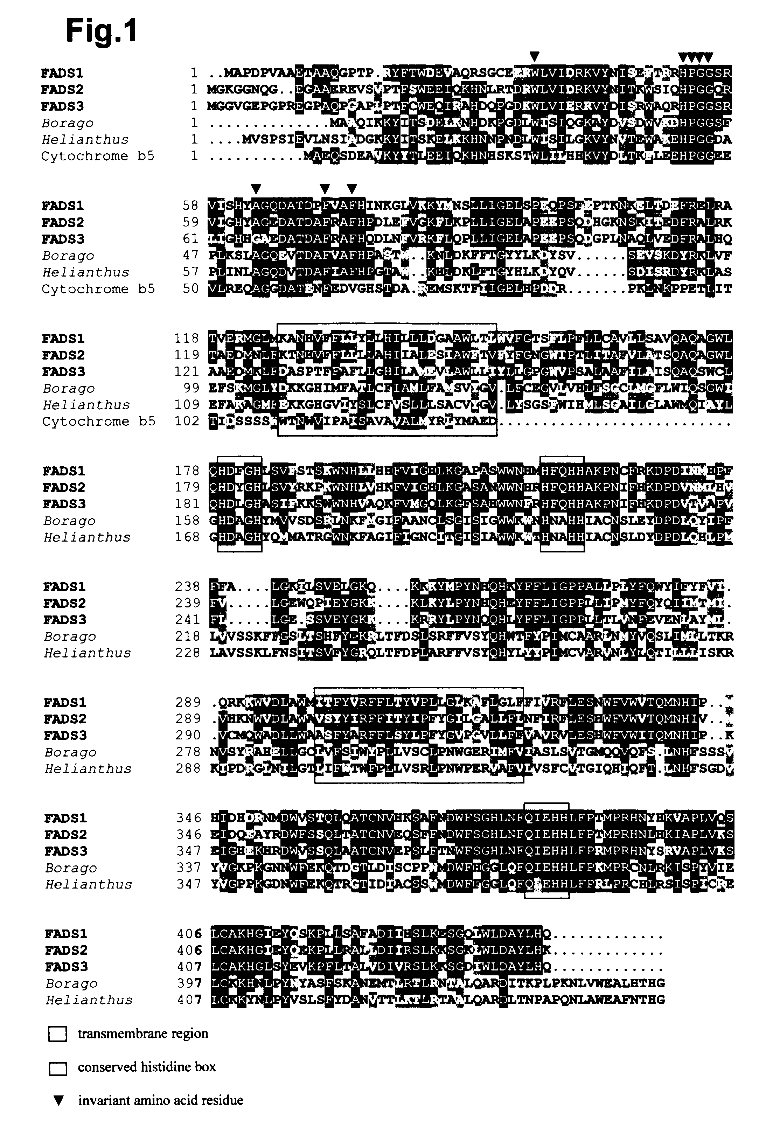

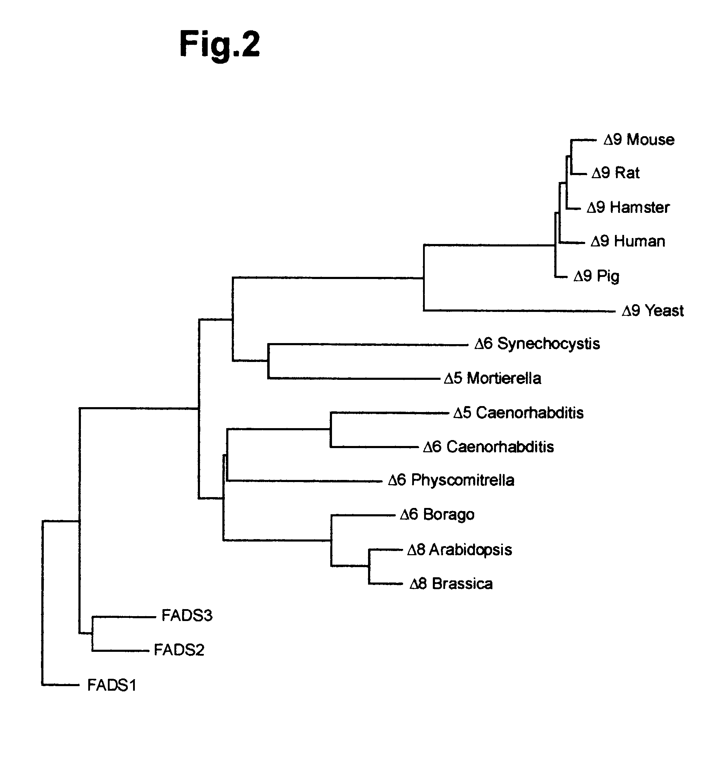

Amino acid identity of FADS1, FADS2 and FADS3 to known desaturases (e.g. from *Arabidopsis thaliana, Brassica napus, Synechocystis spec., Borago officinalis, Helianthus annuus, Saccharomyces cerevisiae* and *Caenorhabditiselegans*) is restricted to the respective carboxy terminal regions (amino acid positions 260 to 422) revealing an overall sequence identity of approximately 27%. Interestingly, the respective amino-termini of the three novel proteins demonstrate similarities to cytochrome b5 (amino acid positions 4 to 75; Fig. 1). Cytochrome b5 is a small hemoprotein and functions as an intermediate donor in a number of oxidation/reduction reactions including e.g. the NADH-dependent Δ9 stearoyl-CoA desaturation (Strittmatter et al. 1974) or the Δ5 desaturation in cholesterol biosynthesis (Reddy et al. 1977). From the amino acid alignments we conclude that FADS1, FADS2 and FADS3 are fusion proteins consisting of a N-terminal cytochrome b5 and a C-terminal desaturase-like enzyme. From a functional point of view, this fusion of two activities may increase the efficiency of electron transport required for desaturation by covalently bringing together the presumed electron donor (cytochrome b5) and its putative acceptor (desaturase-like enzyme). Other heme fusion proteins containing the cytochrome b5 domain have been identified and represent a superfamily of fused proteins (Guiard and Lederer 1979). Besides others this superfamily includes the yeast flavocytochrome b₂, sulfite oxidase, nitrate reductase, the yeast Δ9 acyl-CoA desaturase and more recently the sunflower cytochrome b5-desaturase fusion protein (Sperling et al. 1995). The three novel desaturase-like enzymes reported herein, FADS1, FADS2 and FADS3, can be added to the growing list of members of this superfamily of fused proteins (Fig. 2).

### Summary of the invention

The eukaryotic fatty acid desaturases represent a group of iron-containing enzymes that catalyze NAD(P)H- and O₂-dependent introduction of double bonds into fatty acyl chains. Impairment of desaturase activities has been implicated in a variety of human conditions including liver disease, coronary artery disease and cancer. With the present invention we are providing three isolated human cDNA molecules that encode three novel members of a cytochrome-b5-containing fusion protein with similarity to plant and lower animal desaturase enzymes, termed fatty acid desaturase-1 (FADS1) (represented by Fig. 3 and SEQ ID NO. 1), fatty acid desaturase-2 (FADS2) (represented by Fig. 4 and SEQ ID NO. 2) and fatty acid desaturase-3 (FADS3) (represented by Fig. 5 and SEQ ID NO. 3).

### FADS1 protein

MAPDPVAAETAAQGPTPRYFTWDEVAQRSGCEERWLVIDRKVYNISEFTRRHPGGS RVISHYAGQDATDPFVAFHINKGLVKKYMNSLLIGELSPEQPSFEPTKNKELTDEFREL RATVERMGLMKANHVFFLLYLLHILLLDGAAWLTLWVFGTSFLPFLLCAVLLSAVQAQA GWLQHDFGHLSVFSTSKWNHLLHHFVIGHLKGAPASWWNHMHFQHHAKPNCFRKD PDINMHPFFFALGKILSVELGKQKKKYMPYNHQHKYFFLIGPPALLPLYFQWYIFYFVIQ RKKWVDLAWMITFYVRFFLTYVPLLGLKAFLGLFFIVRFLESNWFVWVTQMNHIPMHID HDRNMDWVSTQLQATCNVHKSAFNDWFSGHLNFQIEHHLFPTMPRHNYHKVAPLVQ SLCAKHGIEYQSKPLLSAFADIIHSLKESGQLWLDAYLHQ

### FADS2 protein

MGKGGNQGEGAAEREVSVPTFSWEEIQKHNLRTDRWLVIDRKVYNITKWSIQHPGG QRVIGHYAGEDATDAFRAFHPDLEFVGKFLKPLLIGELAPEEPSQDHGKNSKITEDFRA LRKTAEDMNLFKTNHVFFLLLLAHIIALESIAWFTVFYFGNGWIPTLITAFVLATSQAQAG WLQHDYGHLSWRKPKWNHLVHKFVIGHLKGASANWWNHRHFQHHAKPNIFHKDPD VNMLHVFVLGEWQPIEYGKKKLKYLPYNHQHEYFFLIGPPLLIPMYFQYQIIMTMIVHKN WVDLAWAVSYYIRFFITYIPFYGILGALLFLNFIRFLESHWFVdNTQMNHIVMEIDQEAY RDWFSSQLTATCNVEQSFFNDWFSGHLNFQIEHHLFPTMPRHNLHKIAPLVKSLCAK HGIEYQEKPLLRALLDIIRSLKKSGKLWLDAYLHK

### FADS3 protein

MGGVGEPGPREGPAQPGAPLPTFCWEQIRAHDQPGDKWLVIERRVYDISRWAQRHP GGSRLIGHHGAEDATDAFRAFHQDLNFVRKFLQPLLIGELAPEEPSQDGPLNAQLVED FRALHQAAEDMKLFDASPTFFAFLLGHILAMEVLAWLLIYLLGPGWVPSALAAFILAISQ AQSWCLQHDLGHASIFKKSWWNHVAQKFVMGQLKGFSAHWWNFRHFQHHAKPNIF HKDPDVTVAPVFLLGESSVEYGKKKRRYLPYNQQHLYFFLIGPPLLTLVNFEVENLAY MLVCMQWADLLWAASFYARFFLSYLPFYGVPGVLLFFVAVRVLESHWFVWITQMNHI PKEIGHEKHRDWVSSQLAATCNVEPSLFTNWFSGHLNFQIEHHLFPRMPRHNYSRVA PLVKSLCAKHGLSYEVKPFLTALVDIVRSLKKSGDIWLDAYLHQ

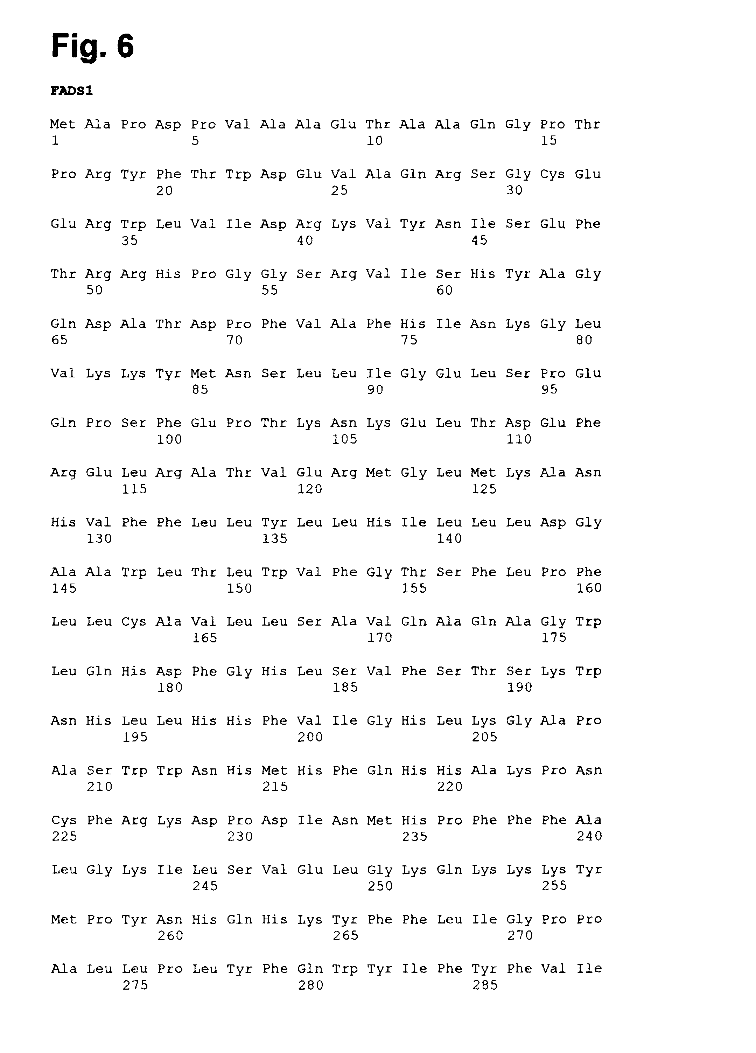

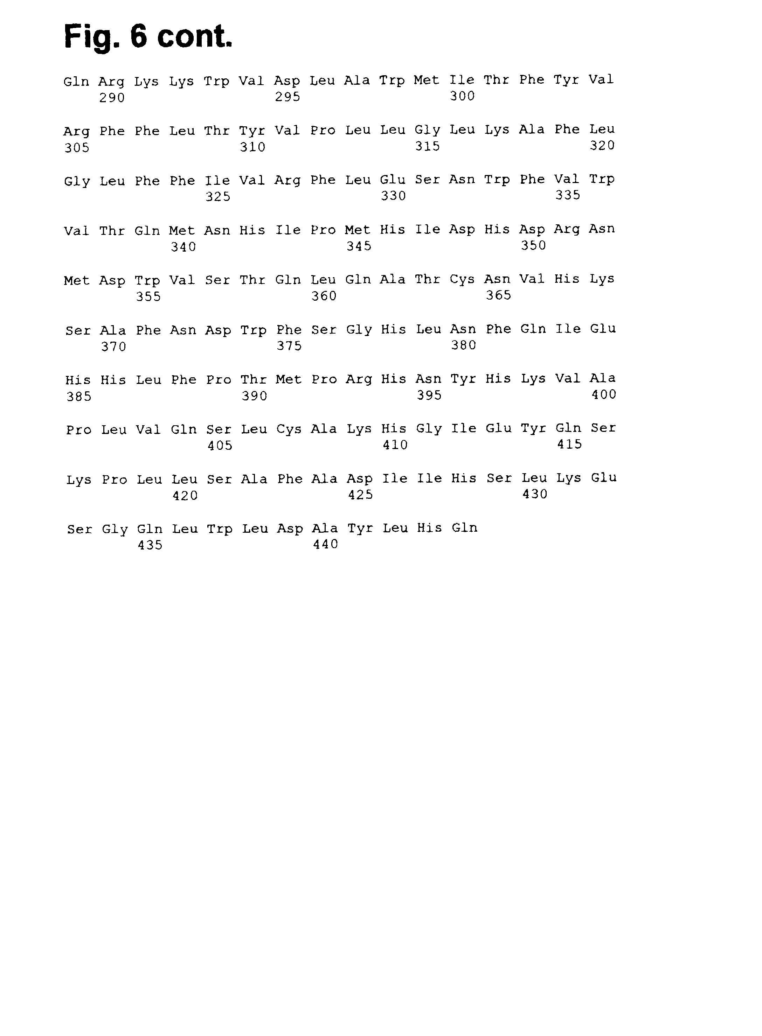

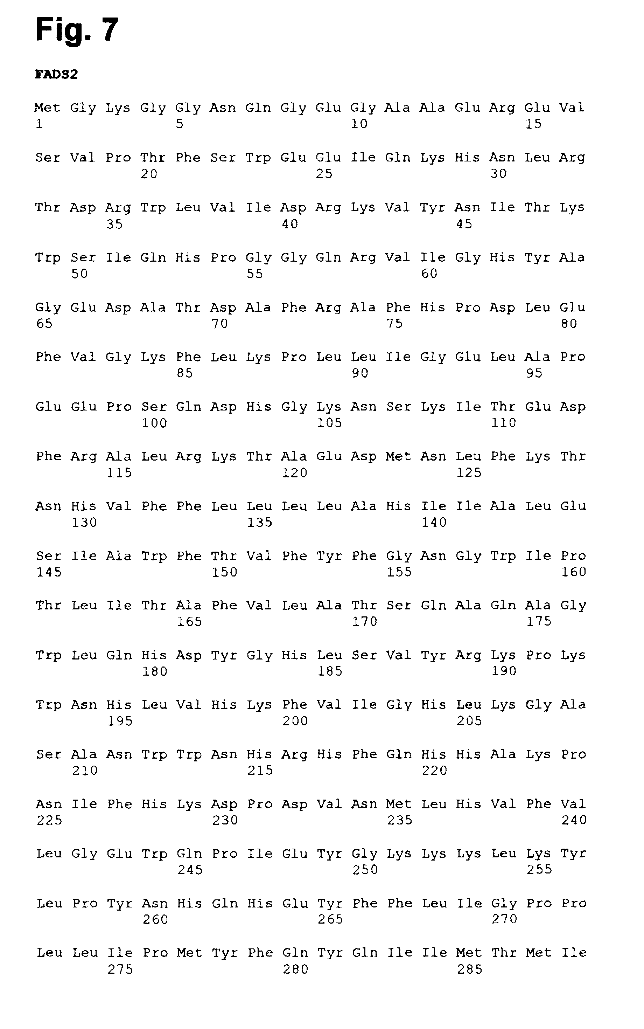

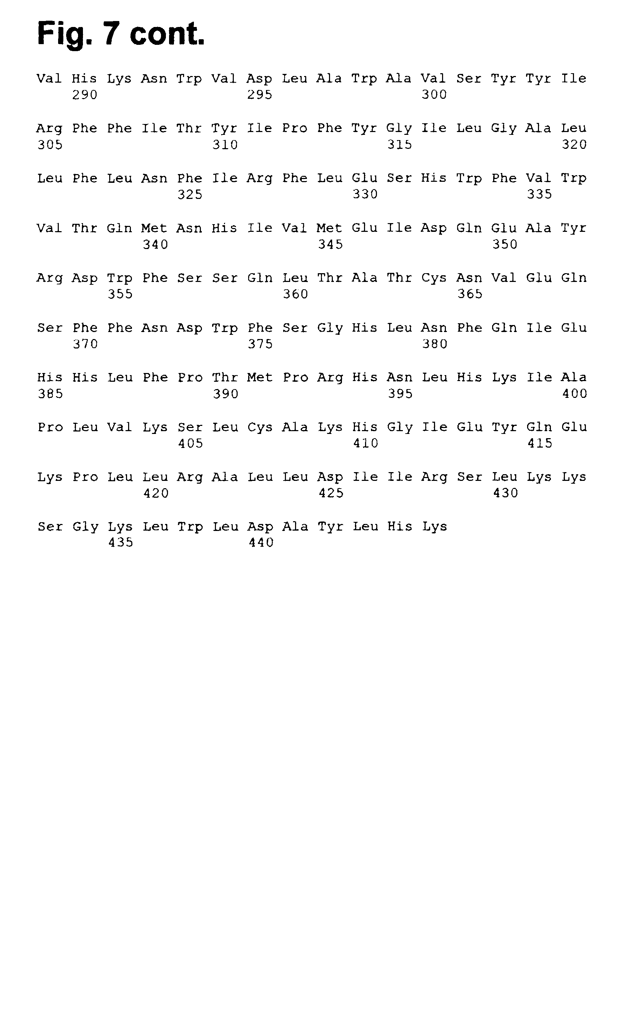

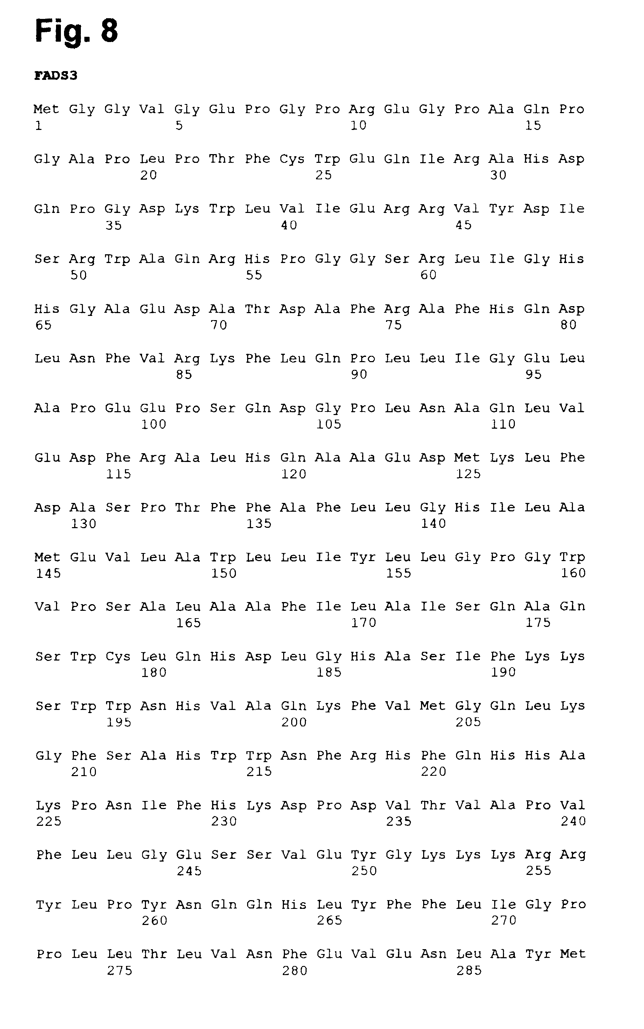

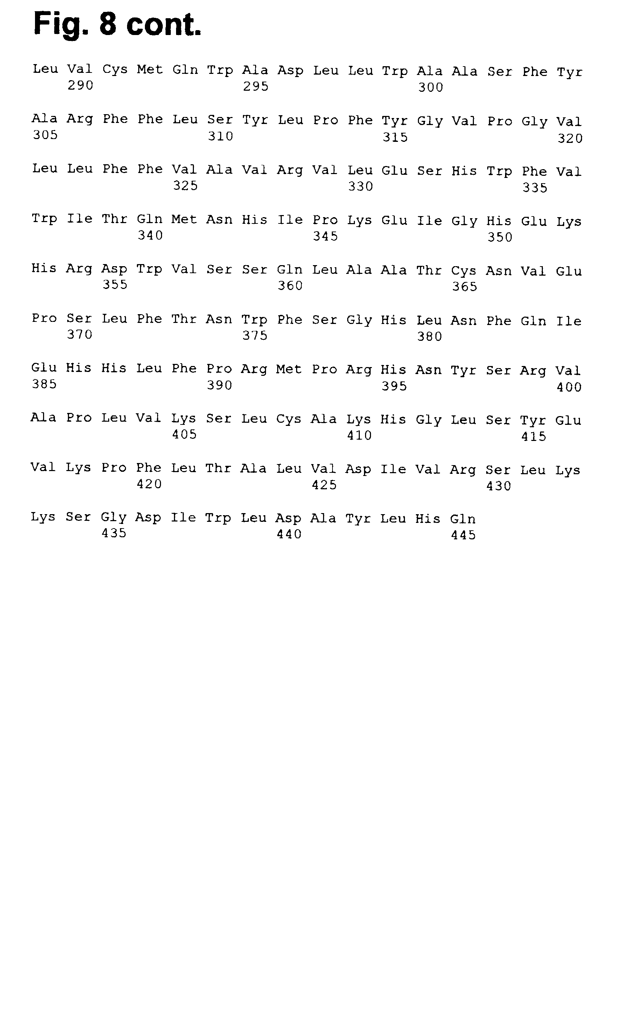

Studies to clarify the specificity and the subcellular location of these ubiquitiously expressed fusion proteins are in progress. Also, the detailed cellular functions and dysfunctions of the desaturase-like domains are being investigated in appropriate cellular and animal systems. This will address the question whether and to which extent these novel enzymes are involved in human disease. The invention encompasses the three cDNA molecules, FADS1, FADS2, and FADS3, the nucleotide sequence of these cDNAs, and the putative amino acid sequences of the FADS1 (represented by Fig. 6 and SEQ ID NO. 4), FADS2 (represented by Fig. 7 and SEQ ID NO. 5), and FADS3 represented by Fig. 8 and SEQ ID NO. 6) proteins.

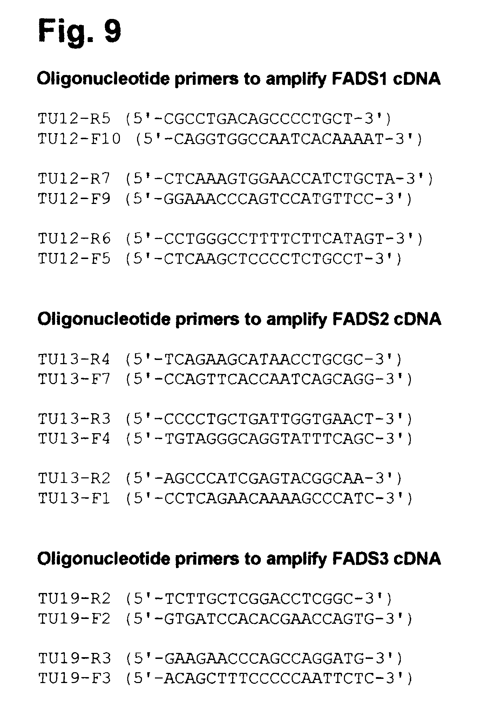

Also comprehended by this invention are oligonucleotide primers comprising the cDNA molecule or its complementary strand allowing the amplification of FADS1 (represented by Fig. 9 and SEQ ID NOS. 7-12), FADS2 (represented by Fig. 9 and SEQ ID NOS. 13-18), and FADS3 (represented by Fig. 9 and SEQ ID NOS. 19-22), by the reverse transcriptase polymerase chain reaction (RT-PCR). Such primers are particularly useful and will provide researchers and physicians with an enhanced ability to assess the role of FADS1, FADS2, and FADS3 in human disease. The present invention also relates to methods of screening for and detection of FADS1, FADS2, and FADS3 mutation carriers including prenatal FADS1, FADS2, and FADS3 screening and diagnosis.

Having provided the isolated human FADS1, FADS2, and FADS3 cDNA sequences, also comprehended by this invention are the FADS1, FADS2, and FADS3 proteins, and derivatives thereof, in aspects of diagnosis and treatment of human disease. Finally, the invention pertains to proteins which comprise the same or substantially the same amino acid sequence (at least 200 amino acids) as that represented by Figs. 6, 7, 8 and SEQ ID NOS. 4, 5, 6 or a variant of the amino acid sequences having a deletion, addition or substitution of 1 to 10 amino acids, or its salt.

Another aspect of the invention is the use of the FADS1, FADS2, and FADS3 proteins as a target for drug and gene therapy in the treatment of human disease. This includes the generation and utilization of FADS1, FADS2, and FADS3-targeted animal models (knock-in, knock-out, transgenic animals) and anti-FADS1, -FADS2, and -FADS3 antibodies that specifically detect the FADS1, FADS2, and FADS3 proteins, respectively.

The foregoing and other features and advantages of the invention will become more apparent from the following detailed description and accompanying drawings.

One aspect of the invention are the isolated cDNAs selected from the group consisting of:
(a) a polynucleotide having at least a 65 % homology, preferably at least a 80 % homology with a polynucleotide encoding a polypeptide selected from the group consisting of the polypeptides of SEQ ID NO: 4, SEQ ID NO: 5 and SEQ ID NO: 6;
(b) a polynucleotide having at least a 65 % homology, preferably at least a 80 % homology with a polynucleotide which by virtue of the redundancy of the genetic code, encodes the same polypeptide selected from the group consisting of the polypeptides of SEQ ID NO: 4, SEQ ID NO: 5 and SEQ ID NO: 6;
(c) a DNA molecule capable of hybridization under stringent conditions to a DNA molecule according to (a) or (b);
(d) a polynucleotide which is complementary to the polynucleotide of (a), (b) or (c); and
(e) a oligonucleotide comprising at least 15 consecutive nucleotides of the polynucleotide of (a), (b), (c) or (d)
(including DNAs which are synonymous to the DNAs of (a), (b), (c), (d) and (e) due to the degeneracy of the genetic code)
especially isolated cDNAs selected from the group consisting of:
(a) a polynucleotide having at least a 65 % homology, preferably at least a 80 % homology with a polynucleotide sequence selected from the group consisting of the polynucleotides of SEQ ID NO: 1, SEQ ID NO: 2 and SEQ ID NO: 3;
(b) a DNA molecule capable of hybridization under stringent conditions to a DNA molecule according to (a);
(c) a polynucleotide which is complementary to the polynucleotide of (a) or (b);
(d) a oligonucleotide comprising at least 15 consecutive nucleotides of the polynucleotide of (a), (b) or (c); and
(e) a DNA which is synonymous to the DNAs of (a), (b), (c) or (d) due to the degeneracy of the genetic code.

In the scope of the invention are polynucleotides having a polynucleotide encoding a polypeptide selected from the group consisting of the polypeptides of SEQ ID NO: 1, SEQ ID NO: 2 and SEQ ID NO: 3 and polynucleotides having a polynucleotide sequence selected from the group consisting of the polynucleotides of SEQ ID NO: 4, SEQ ID NO: 5 and SEQ ID NO: 6, but DNAs comprising a nucleotide sequence with at least a 65 % homology with these nucleotide sequences is also within the scope of the invention.

Furthermore within the scope of the invention are:

A recombinant vector comprising the disclosed DNA molecules.

Transgenic host cells such as COS7, fibroblast cell lines or any other tissue-specific cell lines, as well as a transgenetic host cell tranformed by the DNA or the vector, a corresponding transgenetic organism or a corresponding transgenetic knock-in or knock-out animal model.

Polypeptides and corresponding proteins comprising at least 65 %, preferably 85 %, especially 100 % of a polypeptide sequence selected from the group consisting of the polypeptides of SEQ ID NO: 1, SEQ ID NO: 2 and SEQ ID NO: 3; polypeptides comprising a polypeptide sequence with at least a 65 % homology with the said polypeptides; peptides comprising at least 15, preferably 30, especially 60 consecutive amino acids of the said polypeptides; and polypeptides having substantially the same amino acid sequence as the said polypeptides, or having a variant of the amino acid sequence of the polypeptides with a deletion, addition or substitution of 1 to 10 amino acids. The salts of the peptides and proteins are also within the scope of the invention.

A process for preparing the proteins which comprises cultivating the transformants to form the proteins.

A method of screening for modulators in well known assays using constructs such as FADS1, FADS2, and FADS3 promoter luciferase or green fluorescent protein hybrids or screening for interacting proteins or factors using state of the art technologies like the interaction trap technology to screen for interacting substances of FADS1, FADS2, and FADS3 or isolated domains of FADS1, FADS2, and FADS3.

### A method of screening chemical libraries comprising transformed cell lines

A compound which alters 1 reacts with at least one epitope of the proteins and which is obtained by screening methods utilizing the FADS1, FADS2, and FADS3 cDNAs or protein molecules.

Use of antibodies against the FADS1, FADS2, and FADS3 proteins for diagnostic or therapeutic purposes.

A pharmaceutical composition comprising as an effective component of the proteins or a partial peptide of the proteins, and a pharmaceutically acceptable carrier or diluent.

The term "knock-out animal" as used herein is intended to describe an animal containing a gene which has been modified by homologous recombination. The homologous recombination event may completely disrupt the gene such that a functional gene product can no longer be produced (hence the name "knock-out") or the homologous recombination event may modify the gene such that an altered, although still functional, gene product is produced.

The term "knock-in" as used herein is intended to describe a variation of gene targeting that uses homologous recombination but allows expression of added genetic sequences in place of the endogenous gene. This approach allows the test of more subtle mutations than is allowed by a simple knock-out.

The term "epitope" describes a region on a macromolecule which is recognized by an antibody. Frequently it is in a short region of primary sequence in a protein and it is generally about 5 to 12 amino acids long (the size of the antigen binding site on an antibody). Carbohydrates, nucleic acids and other macromolecules may be antigens and have epitopes.

### Detailed Description of the Invention

### Materials and Methods

Isolation of the FADS1 and FADS2 cDNAs cDNA fragments corresponding to FADS1 and FADS2 were identified by direct cDNA selection. The cDNA selection was performed essentially as described (Rommens et al. 1993) with only minor modifications. Briefly, total RNA was prepared from human retina and from established human retinal pigment epithelium cell line ARPE-19 (Dunn et al. 1996). Prior to the use as templates for cDNA synthesis the isolated RNAs were separated on a 1.2% agarose gel in the presence of 3-(*N*-morpholino)propanesulfonic acid (MOPS) and formaldehyde to check their integrity (Sambrook *et al.,* 1989).

RNAs were reverse transcribed using the SUPERSCRIPT™ preamplification system for first strand cDNA synthesis (Gibco, BRL) and the RXGT₁₂ oligonucleotide primer (5'-CGG AAT TCT CGA GAT CTT TTT TTT TTT TT-3'). After poly(A)-tailing with terminal transferase (United States Biochemical, USB), a cDNA pool was generated by RXGT₁₂-primed PCR at 94°C for 1 min; 2 cycles of 94°C, 30 sec; 37°C, 1 min, 72°C, 2 min followed by 22 cycles of 94°C, 30 sec; 58°C, 30 sec and 72°C, 2 min. Prior to hybridization the cDNA pools were pre-annealed to Cₒt-1 DNA (Gibco, BRL) enriched with sonicated LINE1 sequences.

Genomic PAC clones for cDNA selection were derived from 11q12-q13.1, a region known to contain the gene underlying Best's vitelliform macular dystrophy (Stöhr et al. 1998). The assembly and orientation of the clones have been described previously (Cooper et al. 1997). Inserts from PAC clones dJ465G21 and dJ139E20 (∼1 µg) were isolated by Notl digestion, purified using QIAEXII agarose gel extraction beads (Qiagen) and immobilized on Hybond-N+ membrane filters with an average concentration of 60 ng/mm². The insert filters were subjected to two consecutive rounds of hybridization with a starting mixture of 20 µg of retina and ARPE-19 derived cDNAs. Hybridization time was four days at 58°C in Church hybridization buffer (Church and Gilbert 1984). Filters were washed three times in 2 x SSC/0.1% SDS at room temperature, once each in 0.5 x SSC/0.1% SDS, 0.2 x SSC/0.1% SDS and 0.2 x SSC/0.05% SDS (all at 58°C). A final wash was in 2 x SSC. cDNAs were eluted in distilled H₂O by incubating for 10 min at 98°C and reamplified by PCR using the RXGT₁₂ oligonudeotide primer. Four µg of the reamplified cDNAs were used for a second round of hybridization. After two rounds of selection the cDNAs were amplified using the RXGT₁₂ oligonucleotide primer, digested with EcoRI and cloned into the EcoRI site of pBluescript (Stratagene).

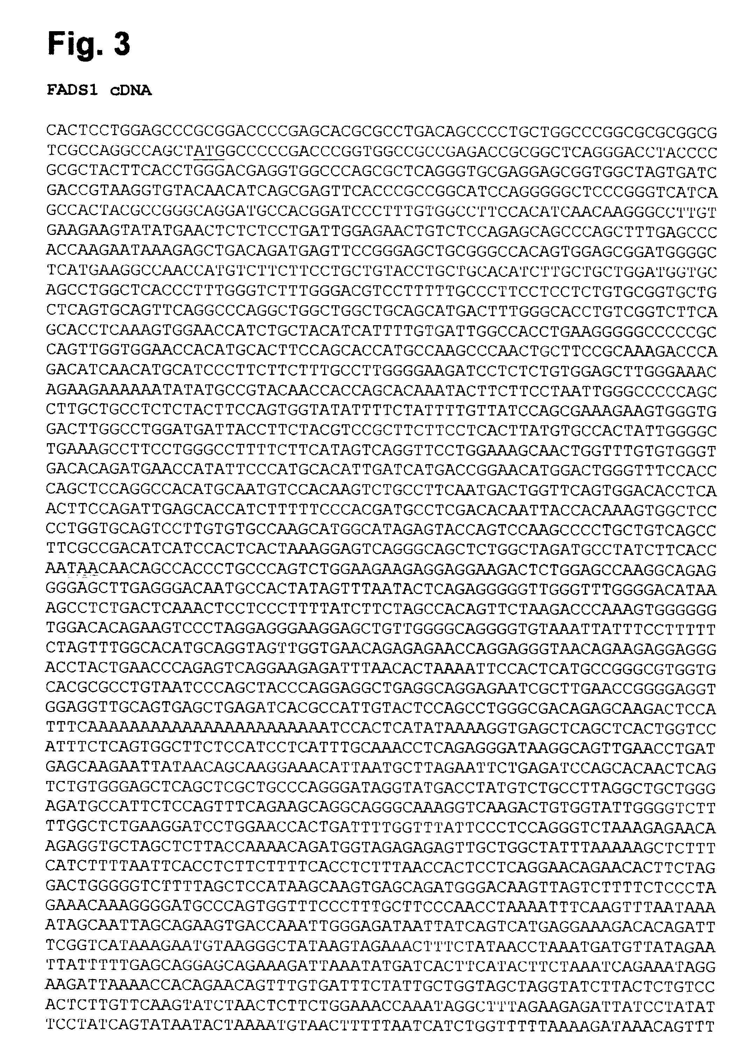

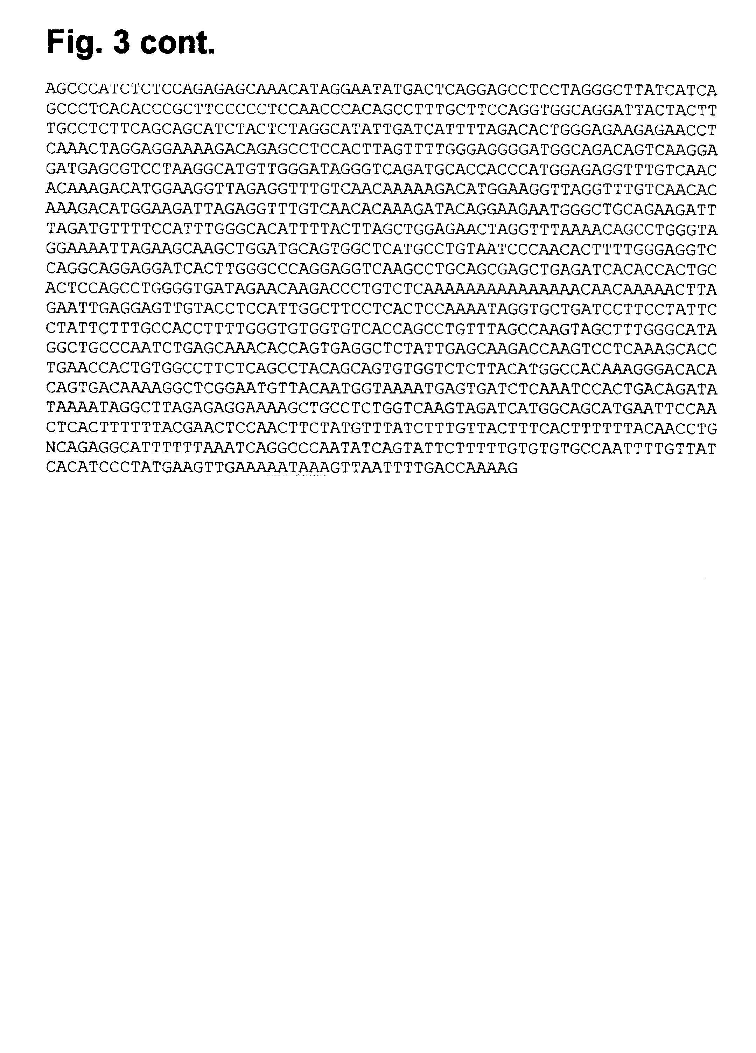

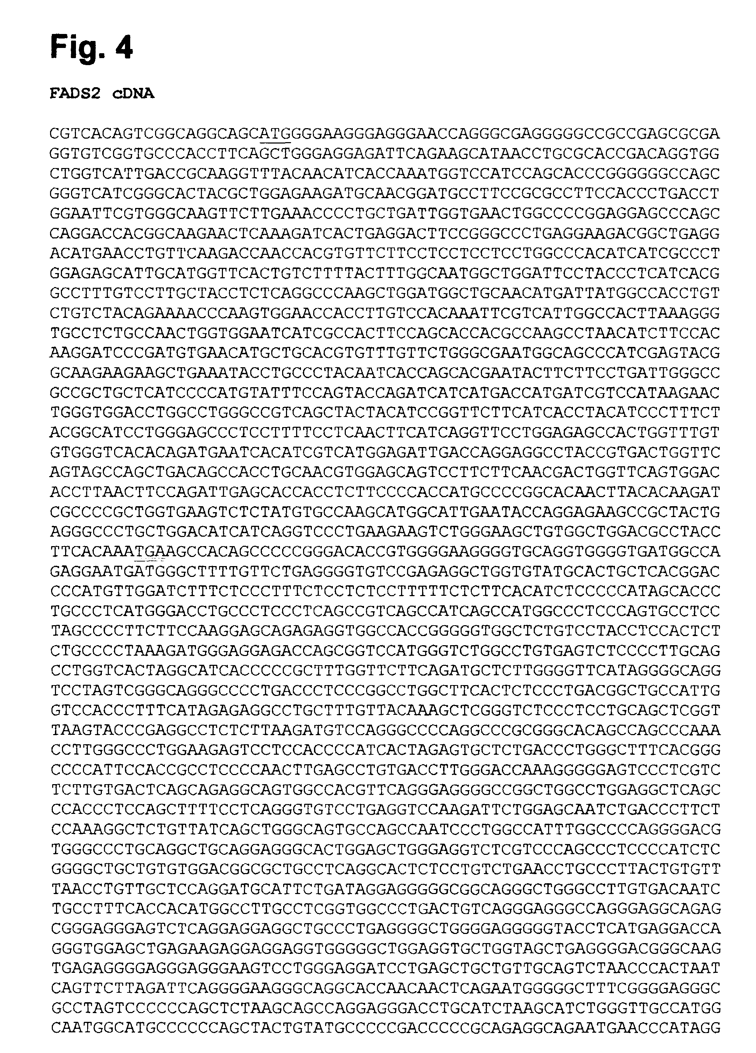

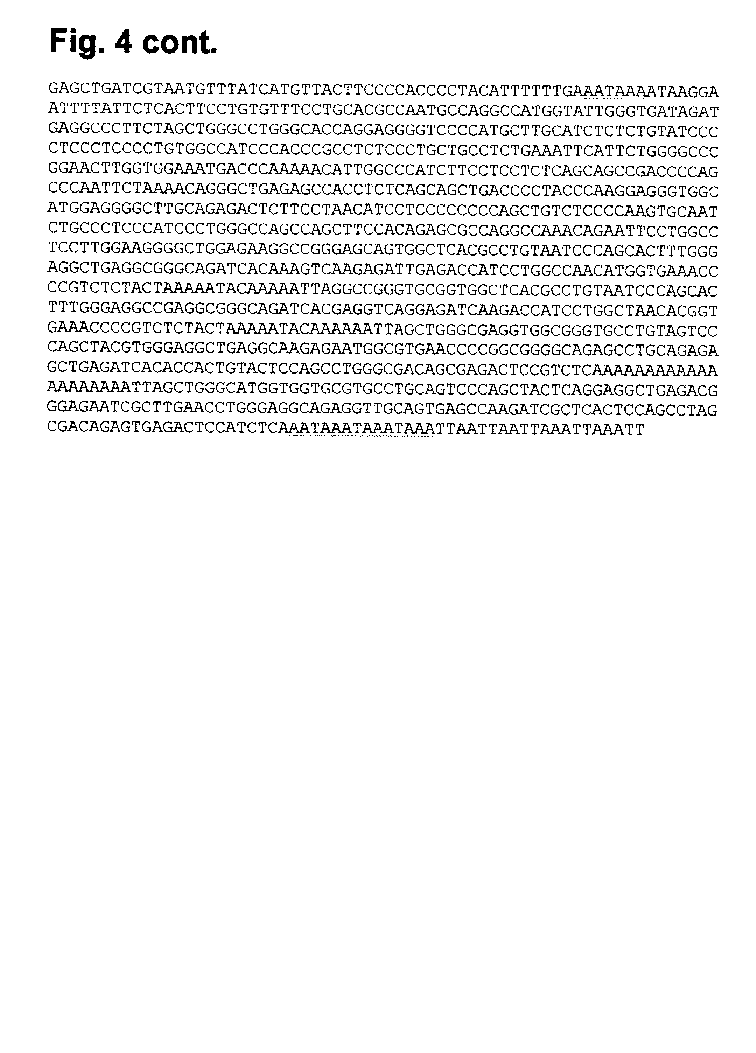

The selected cDNAs represent segments of the 3'-untranslated region (3'-UTR) of FADS1 (clone IVC4 at FADS1 nucleotide position 3793-4204; clone IVB7 at nucleotide position 3132-3609; done VIIC6 at nucleotide position 2077-2317) (Fig. 3) and of the 3' UTR/coding sequence of FADS2 (done IVB8 at FADS2 nucleotide position 2626-3009; clone TUK8-4B at nucleotide position 753-1508) (Fig. 4).

Using the selected clone sequences extensive dbEST database searches were conducted and revealed a large number of additional overlapping expressed sequence tags (ESTs). More than 100 ESTs (e.g. zk09h08, EST177650, yb28c03, ym29b05, yx67h05) were assembled to an overlapping EST contig representing FADS1. The assembled EST sequences contain an open reading frame (ORF) of 1410 bp, with a first potential in-frame translation initiation codon, ATG, starting 79 nucleotides downstream the most 5'end of EST clone zk09h08.r1 (GenBank acc. no. AA029030) (Fig. 1a). A consensus polyadenylation signal, AAUAAA, was identified at nucleotide position 4.182. The mature protein predicted from the ORF consists of 444 amino acid residues resulting in a calculated molecular mass of 52.0 kDa (Fig. 6).

Another 30 overlapping ESTs (e.g. cp2485.seq, HSC2EA121, EST06759, ym42c04, nc08c05) were found facilitating the assembly of the FADS2 cDNA. The assembled EST sequences contain an open reading frame (ORF) of 1352 bp, with a first potential in-frame translation initiation codon, ATG, starting 21 nucleotides downstream the most 5'end of EST done ub64e01.r1 (GenBank acc. no. Al036465) (Fig. 4). Consensus polyadenylation signals were predicted at nucleotide positions 2.996 and 4.056. The mature FADS2 protein predicted from the ORF consists of 444 amino acid residues resulting in a calculated molecular mass of 52.3 kDa (Fig. 7). Amino acid sequence identity between FADS1 and FADS2 is 62%.

### Isolation of the FADS3 cDNA

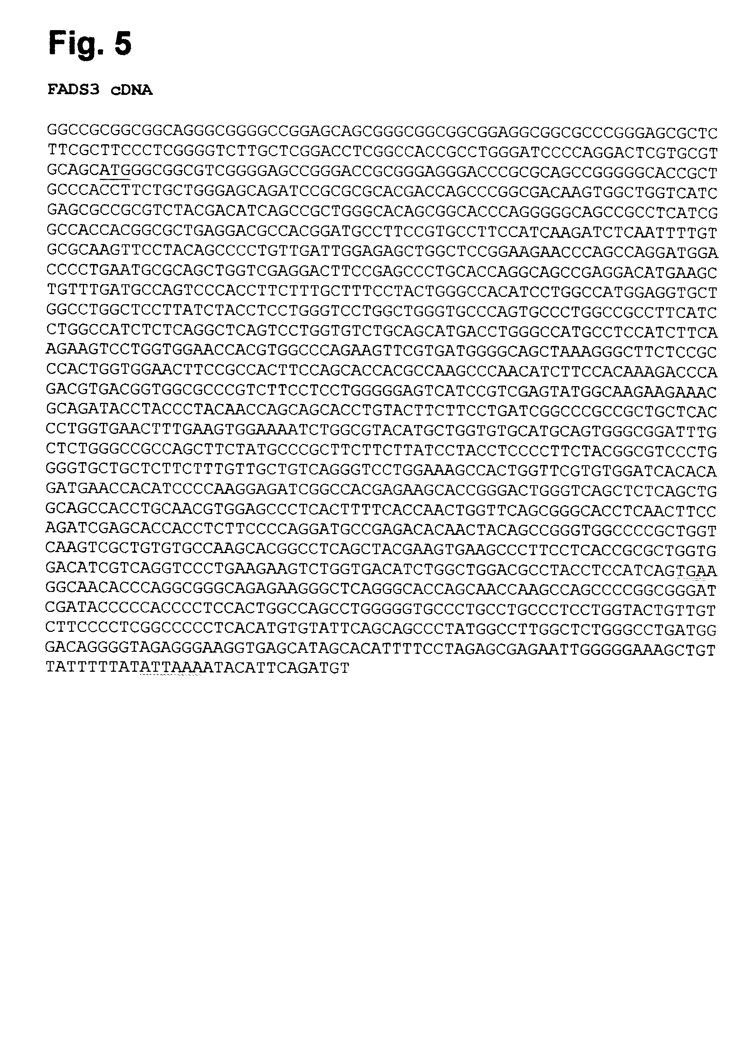

Additional 30 human EST clones were available to assemble a third individual cDNA, termed FADS3 (e.g. zs84e06, zs84e05, nq23f05, ya49a19, zs86h09). The existence of a third member of the FADS family was confirmed by PCR mapping of FADS1-, FADS2- and FADS3-specific 3'-UTR fragments revealing three distinct gene loci within a 1.4 Mb PAC contig in 11q12-q13.1 (Cooper *et al.,* 1997). The assembled EST sequences contain an open reading frame (ORF) of 1468 bp, with a first potential in-frame translation initiation codon, ATG, starting 134 nucleotides downstream the most 5'end of EST clone qa99d06.s1 (GenBank acc. no. Al123992) (Fig. 5). The mature protein predicted from the ORF consists of 445 amino acid residues resulting in a calculated molecular mass of 51.2 kDa (Fig. 8). The 3'-UTR of the FADS3 cDNA is represented by several EST clones (e.g. zs86h09.s1, AA279632). A potential polyadenylation signal, AUUAAA, is present at cDNA nucleotide position 1.757 and may be functional as AUUAAA is the most common natural variant of the consensus polyadenylation signal AAUAAA (Fig. 5) (Sheets *et al.,* 1990).

Amino acid sequence identities between FADS1 and FADS3 as well as between FADS2 and FADS3 are 52% and 63%, respectively. All EST sequences in the dbEST databases could be aligned to one of the three cDNAs, FADS1, FADS2, and FADS3. This suggests that there are no additional members of the FADS family in the human genome.

### Northern blot analysis

Northern blot analysis was performed either with total RNA isolated using the guanidinium thiocyanate method (Chomczynski and Sacchi 1987) or with commercially available multiple tissue Northern (MTN) blots purchased from Clontech Laboratories Inc. (Palo Alto, CA). Each lane of the total RNA blot contained 12 µg of total RNA from lung, cerebellum, uterus, retina, liver, heart, RPE cell line ARPE-19, RPE tissue, lymphocytes and was electrophoretically separated in the presence of formaldehyde. The MTN blots were prepared from poly(A)⁺ RNA isolated from human heart, brain, placenta, lung, liver, skeletal muscle, kidney and pancreas. Inserts of clones IVC4, IVB7 (FADS1), IVB8 (FADS2) and of the 362 bp PCR product F3/R (5'-ACAGCTTTCCCCCAATTCTC-3'/5'-GGCCTCAGCTACGAAGTGAAG-3') (FADS3) derived from the 3'-UTRs of the respective genes were used for filter hybridization at 65°C in 0.5 mM sodium phosphate buffer, pH 7.2; 7% SDS, 1 mM EDTA at 65°C (Church and Gilbert 1984).

The three genes are ubiquitiously expressed and appear to have similar expression levels in all tissues analyzed. FADS1 revealed a transcript size of 4.0 kb while FADS2 revealed a similar sized transcript of 4.0 kb in addition to a smaller transcript of approximately 3.1 kb. The two FADS2 variants may be due to differential usage of polyadenylation signals (see above). Finally, FADS3 is represented by two transcripts of 1.75 kb and 1.25 kb in size. While the former is in agreement with the usage of the variant polyadenylation signal identified at position 1738 of the cDNA, the small size of the latter transcript can not be explained at present and it does not appear to be due to a differential usage of polyadenylation signals. Possibly, differential splicing and/or exon skipping may be involved in the generation of the variant transcript. However, there is no evidence from cDNA cloning or EST contig assembly to support this possibility.

### Comparison with other desaturases

Local sequence alignments of the deduced amino acid sequences of FADS1, FADS2, and FADS3 with known proteins or protein motifs were done using SwissProt (http://www.ncbi.nlm.nih.gov/cgi-bin/Blast/nph-blast?Jform=0) and the BLASTP and BEAUTY programs at Baylor College of Medicine (http://dot.imgen.bcm.tmc.edu:9331/seq-search/protein-search.html). Amino acid sequence alignments were performed using the CLUSTALW multiple alignment program at http://pbil.ibcp.fr/NPSA/npsa_clustalw.html. Phylogenetic tree assembly was done using the TREECON software Version 1.3b available at http://bioc-www.uia.ac.be/u/yvdp/index.html.

Overall amino acid identities to known desaturases were found to be in the range of 22% - 27% (Fig. 1). Phylogenetic tree construction revealed a genetic relationship of FADS1, FADS2, and FADS3 to the Δ5-, Δ6- and Δ8-desaturases with some distance to the Δ9-desaturases (Fig. 2). From these analyses it becomes obvious that sequence identity by itself is not a predictor of a specific desaturase activity. For example, Δ5- and Δ6-desaturases from C. elegans demonstrate a higher sequence identity to each other than to the Δ6-desaturases from other species. We therefore conclude that based on simple sequence comparisons it is not feasible to determine the specific functions of FADS1, FADS2, and FADS3. This will be done by transgene expression of the three desaturases combined with gas chromatograpy-mass spectometry.

Hydropathy plots of FADS1, FADS2, and FADS3 indicate two hydrophiobic sequences predicted to represent transmembrane-spanning domains similar to other desaturases identified thus far (Fig. 1) (reviewed in Sperling et al. 1995).

### cDNA amplification of FADS1, FADS2, and FADS3

The coding sequences of the three genes are amplified in overlapping fragments by performing RT-PCR using oligonucleotide primer pairs derived from the respective cDNA sequences:

### (1) FADS1 (Fig. 9 and SEQ ID NOS. 7-12)

Sense primer TU12-R5 (5'-CGCCTGACAGCCCCTGCT-3') at cDNA position 31-48 in combination with antisense primer TU12-F10 (5'-CAGGTGGCCAATCACAAAAT-3') at cDNA position 671-690 results in a product of 660 bp; sense primer TU12-R7 (5'-CTCAAAGTGGAACCATCTGCTA-3') at cDNA position 645-666 in combination with antisense primer TU12-F9 (5'-GGAAACCCAGTCCATGTTCC-3') at cDNA position 1130-1149 results in a product of 505 bp; sense primer TU12-R6 (5'-CCTGGGCCTTTTCTTCATAGT-3') at cDNA position 1035-1055 in combination with antisense primer TU12-F5 (5'-CTCAAGCTCCCCTCTGCCT-3') at cDNA position 1465-1483 results in a product of 449 bp.

### (2) FADS2 (Fig. 9 and SEQ ID NOS. 13-18)

Sense primer TU13-R4 (5'-TCAGAAGCATAACCTGCGC-3') at cDNA position 98-116 in combination with antisense primer TU13-F7 (5'-CCAGTTCACCAATCAGCAGG-3') at cDNA position 284-303 results in a product of 206 bp; sense primer TU13-R3 (5'-CCCCTGCTGATTGGTGAACT-3') at cDNA position 282-301 in combination with antisense primer TU13-F4 (5'-TGTAGGGCAGGTATTTCAGC -3') at cDNA position 779-798 results in a product of 517 bp; sense primer TU13-R2 (5'-AGCCCATCGAGTACGGCAA-3') at cDNA position 754-772 in combination with antisense primer TU13-F1 (5'-CCTCAGAACAAAAGCCCATC-3') at cDNA position 1416-1435 results in a product of 682 bp.

### (3) FADS3 (Fig. 9 and SEQ ID NOS. 19-22)

Sense primer TU19-R2 (5'-TCTTGCTCGGACCTCGGC-3') at LLCDL3 cDNA position 81-98 in combination with antisense primer TU19-F2 (5'-GTGATCCACACGAACCAGTG-3') at cDNA position 1130-1149 position results in a product of 1069 bp; sense primer TU19-R3 (5'-GAAGAACCCAGCCAGGATG-3') at cDNA position 428-446 in combination with antisense primer TU19-F3 (5'-ACAGCTTTCCCCCAATTCTC-3') at cDNA position 1709-1728 results in a product of 1301 bp.

### Short description of Figures

Fig. 1 Comparison of putative amino acid sequences from FADS1, FADS2, FADS3, Borago officinalis, Helianthus annuus and human cytochrome b5. Arrowheads indicate eight invariant amino acid residues typical for the cytochrome b5 domain. Two potential transmembrane domains are boxed. Three histidine motifs HX₂₍₃₎[XH]H that are conserved within the desaturase family are hatched.
Fig. 2 Phylogenetic tree of fatty acid desaturases.
Fig. 3 (SEQ ID NO. 1) shows the nucleotide sequence of the FADS1 cDNA
Fig. 4 (SEQ ID NO. 2) shows the nucleotide sequence of the FADS2 cDNA
Fig. 5 (SEQ ID NO. 3) shows the nucleotide sequence of the FADS33 cDNA
Fig. 6 (SEQ ID NO. 4) shows the putative amino acid sequence of the predicted FADS1 protein
Fig. 7 (SEQ ID NO. 5) shows the putative amino acid sequence of the predicted FADS2 protein
Fig. 8 (SEQ ID NO. 6) shows the putative amino acid sequence of the predicted FADS3 protein
Fig. 9 (SEQ ID NOS. 7-22) shows the oligonucleotide PCR primers utilized to amplify the FADS1, FADS2, FADS3 cDNA, respectively.

### References

Ariza-Ariza R, Mestanza-Peralta M, Cardiel MH. Omega-3 fatty acids in rheumatoid arthritis: an overview. Semin Arthritis Rheum 27: 366-370 (1998)
Calder PC. Immunoregulatory and anti-inflammatory effects of n-3 polyunsaturated fatty acids. Braz J Med Biol Res 31: 467-490 (1998)
Chi Y, Gupta RK. Alterations in membrane fatty acid unsaturation and chain length in hypertension as observed by 1H NMR spectroscopy. Am J Hypertens 11: 340-348 (1998)
Chomczynski, P. and Sacchi, N. Single-step method of RNA isolation by acid guanidinium thiocyanate-phenol-chloroform extraction. Anal Biochem 162: 156-159 (1987)
Church, G.M., and W. Gilbert. Genomic sequencing. Proc Natl Acad Sci USA 81:1991-1995 (1984)
Cook HW. Fatty acid desaturation and chain elongation in eucaryotes. In: Biochemistry of lipids, lipoproteins and membranes, Vance DE & Vance JE (eds), Elsevier Amsterdam London, New York, pp.141-169 (1991)
Cooper P, Nowak NJ, Higgins MJ, Simpson SA, Stöhr H, Marquardt A, Weber BHF, Gerhard DS, deJong P, Shows TB. A sequence ready high resolution physical map of the Best's macular dystrophy gene region in 11q12-q13. Genomics 41: 185-192 (1997)
Delton-Vandenbrouke I, Grammas P, Anderson RE. Polyunsaturated fatty acid metabolism in retinal and cerebral microvascular endothelial cells. J Lipid Res 38:147-159(1997)
Dunn KC, Aotaki-Keen AE, Putkey FR, Hjelmeland LM. ARPE-19, a human retinal pigment epithelial cell line with differentiated properties. Exp Eye Res 62: 155-169 (1996)
Fan YY, Chapkin RS. Importance of dietary gamma-linolenic acid in human health and nutrition. J Nutr 128: 1411-1414 (1998)
Grattan C, Burton JL, Manku M, Stewart C, Horrobin DF. Essential-fatty-acid metabolites in plasma phospholipids in patients with ichthyosis vulgaris, acne vulgaris and psoriasis. Clin Exp Dermatol 15:174-176 (1990)
Grimble RF, Tappia PS. Modulation of pro-inflammatory cytokine biology by unsaturated fatty acids. Z Ernährungswiss 37 (Suppl 1): 57-65 (1998)
Guiard B, Lederer F. The "cytochrome b5 fold": structure of a novel protein superfamily. J Mol Biol 135: 639-50 (1979)
Hodge L, Salome CM, Hughes JM, Liu-Brennan D, Rimmer J, Allman M, Pang D, Armour C, Woolcock AJ. Effect of dietary intake of omega-3 and omega-6 fatty acids on severity of asthma in children. Eur Respir J 11: 361-536 (1998)
Horrobin DF. Abnormal membrane concentrations of 20 and 22-carbon essential fatty acids: a common link between risk factors and coronary and peripheral vascular disease? Prostaglandins Leukot Essent Fatty Acids 53: 385-396 (1995)
Horrobin DF. Essential fatty acids in clinical dermatology. J Am Acad Dermatol 20: 1045-1053 (1989)
Horrobin DF. Fatty acid metabolism in health and disease: the role of Δ6-desaturase. Am J Clin Nutr 57(5 Suppl): 732S-737S (1993)
James MJ, Cleland LG. Dietary n-3 fatty acids and therapy for rheumatoid arthritis. Semin Arthritis Rheum 27: 85-97 (1997)
Jiang WG, Bryce RP, Horrobin DF, Mansel RE. gamma-Linolenic acid blocks cell cycle progression by regulating phosphorylation of p27kip1 and p57kip2 and their interactions with other cycle regulators in cancer cells. Int J Oncol 13: 611-617 (1998b)
Jiang WG, Hiscox S, Bryce RP, Horrobin DF, Mansel RE. The effects of n-6 polyunsaturated fatty acids on the expression of nm-23 in human cancer cells. Br J Cancer 77: 731-738 (1998a)
Jiang WG, Hiscox S, Hallett MB, Horrobin DF, Mansel RE, Puntis MC. Regulation of the expression of E-cadherin on human cancer cells by gamma-linolenic acid (GLA). Cancer Res 55: 5043-5048 (1995)
Jiang WG, Hiscox S, Horrobin DF, Bryce RP, Mansel RE. Gamma linolenic acid regulates expression of maspin and the motility of cancer cells. Biochem Biophys Res Commun 237: 639-644 (1997b)
Jiang WG, Singhrao SK, Hiscox S, Hallett MB, Bryce RP, Horrobin DF, Puntis MC, Mansel RE. Regulation of desmosomal cell adhesion in human tumor cells by polyunsaturated fatty acids. Clin Exp Metastasis 15: 593-602 (1997a)
Leichsenring M, Kochsiek U, Paul K. (n-6)-Fatty acids in plasma lipids of children with atopic bronchial asthma. Pediatr Allergy Immunol 6: 209-212 (1995)
Moore SA, Yoder E, Murphy S, Dutton GR, Spector AA. Astrocytes, not neurons, produce docosahexaenoic acid (22:6 omega-3) and arachidonic acid (20:4 omega-6). J Neurochem 56: 518-524 (1991)
Mori Y, Murakawa Y, Katoh S, Hata S, Yokoyama J, Tajima N, Ikeda Y, Nobukata H, Ishikawa T, Shibutani Y. Influence of highly purified eicosapentaenoic acid ethyl ester on insulin resistance in the Otsuka Long-Evans Tokushima Fatty rat, a model of spontaneous non-insulin-dependent diabetes mellitus. Metabolism 46: 1458-1464 (1997)
Reddy VV, Kupfer D, Caspi E. Mechanism of C-5 double bond introduction in the biosynthesis of cholesterol by rat liver microsomes. J Biol Chem 252: 2797-2801 (1977)
Rommens JM, Lin B, Hutchinson GB, Andrew SE, Goldberg YP, Glaves ML, Graham R, Lai V, McArthur J, Nasir J, Theilmann J, McDonald H, Kalchman M, Clarke LA, Schappert K, Hayden MR. A transcription map of the region containing the Huntington disease gene, Hum Mol Genet 2: 901-907 (1993)
Russo C, Olivieri O, Girelli D, Guarini P, Pasqualini R, Azzini M, Corrocher R. Increased membrane ratios of metabolite to precursor fatty acid in essential hypertension. Hypertension 29: 1058-1063 (1997)
Sambrook J, Fritsch EF, Maniatis T. Molecular Cloning, A Laboratory Manual, 2nd Edition, Cold Spring Harbor Laboratory Press, USA (1989)
Shanklin J, Whittle E, Fox BG. Eight histidine residues are catalytically essential in a membrane-associated iron enzyme, stearoyl-CoA desaturase, and are conserved in alkane hydroxylase and xylene monooxygenase. Biochem 33: 12787-12794 (1994)
Shanklin J, Whittle EJ, Fox BG. Membrane bound desaturases and hydroxylases: Structure function studies, in *Plant lipid metabolism,* Kader JC, Mazliak P (eds), Kluwer Academic Publishers, Netherlands, pp.18-20 (1995).
Sheets MD, Ogg SC, Wickens MP. Point mutations in AAUAAA and the poly (A) addition site: effects on the accuracy and efficiency of cleavage and polyadenylation in vitro. Nucl Acid Res 18: 5799-5805 (1990)
Singer SJ, Nicolson GL. The fluid mosaic model of the structure of cell membranes. Science 175: 720-731 (1972)
Sperling P, Schmidt H, Heinz E. A cytochrome b5-containing fusion protein similar to plant acyl lipid desaturases. Eur J Biochem 232: 798-805 (1995)
Stöhr H, Marquardt A, Rivera A, Cooper PR, Nowak NJ, Shows TB, Gerhard DS, Weber BHF. A gene map of the Best's vitelliform macular dystrophy region in chromosome 11q12-q13.1. Genome Res 8: 48-56 (1998)
Strittmatter P, Spatz L, Corcoran D, Rogers MJ, Setlow B, Redline R. Purification and properties of rat liver microsomal stearyl coenzyme A desaturase. Proc Natl Acad Sci USA 71: 4565-4569 (1974)
Stubbs CD, Smith AD. The modification of mammalian membrane polyunsaturated fatty acid composition in relation to membrane fluidity and function. Biochim Biophys Acta 779: 89-137 (1984)
Villani F, Comazzi R, De Maria P, Galimberti M. Effect of dietary supplementation with polyunsaturated fatty acids on bronchial hyperreactivity in subjects with seasonal asthma. Respiration 65: 265-269 (1998)
Wang N, Anderson RE. Synthesis of docosahexaenoic acid by retina and retinal pigment epithelium. Biochemistry 32: 13703-13709 (1993)

## Claims

1. An isolated cDNA molecule selected from the group consisting of:
(a) a polynucleotide having at least a 65 % homology, preferably at least a 80 % homology with a polynucleotide encoding a polypeptide selected from the group consisting of the polypeptides of SEQ ID NO: 4, SEQ ID NO: 5 and SEQ ID NO: 6;
(b) a polynucleotide having at least a 65 % homology, preferably at least a 80 % homology with a polynucleotide which by virtue of the redundancy of the genetic code, encodes the same polypeptide selected from the group consisting of the polypeptides of SEQ ID NO: 4, SEQ ID NO: 5 and SEQ ID NO: 6;
(c) a DNA molecule capable of hybridization under stringent conditions to a DNA molecule according to (a) or (b);
(d) a polynucleotide which is complementary to the polynucleotide of (a), (b) or (c); and
(e) a oligonucleotide comprising at least 15 consecutive nucleotides of the polynucleotide of (a), (b), (c) or (d).

2. An isolated cDNA molecule selected from the group consisting of:
(a) a polynucleotide having at least a 65 % homology, preferably at least a 80 % homology with a polynucleotide sequence selected from the group consisting of the polynucleotides of SEQ ID NO: 1, SEQ ID NO: 2 and SEQ ID NO: 3;
(b) a DNA molecule capable of hybridization under stringent conditions to a DNA molecule according to (a);
(c) a polynucleotide which is complementary to the polynucleotide of (a) or (b);
(d) a oligonucleotide comprising at least 15 consecutive nucleotides of the polynucleotide of (a), (b) or (c); and
(e) a DNA which is synonymous to the DNAs of (a), (b), (c) or (d) due to the degeneracy of the genetic code.

3. A DNA comprising a nucleotide sequence with at least a 65 % homology with the nucleotide sequences as defined in claim 1 or 2.

4. A recombinant vector comprising the DNA as claimed in any of claims 1 to 3.

5. A transgenic host cell comprising the DNA as claimed in any of claims 1 to 3.

6. A transgenetic host cell transformed by the DNA according to any of claims 1 to 3 or the vector according to claim 4, a corresponding transgenetic organism or a corresponding transgenetic knock-in or knock-out animal model.

7. A polypeptide comprising at least 65 % of a polypeptide sequence selected from the group consisting of the polypeptides of SEQ ID NO: 1, SEQ ID NO: 2 and SEQ ID NO: 3, or its salt.

8. A polypeptide comprising a polypeptide sequence with at least a 85 % homology with the polypeptide sequence as claimed in claim 7, or its salt.

9. A peptide comprising at least 15 consecutive amino acids of the polypeptide as claimed in claim 7, or its salt.

10. A polypeptide having substantially the same amino acid sequence as the polypeptide as claimed in claim 7, or having a variant of the amino acid sequence of the polypeptide as claimed in claim 7 with a deletion, addition or substitution of 1 to 10 amino acids, or its salt.

11. A process for producing a polypeptide comprising expressing from the host cell of claim 5 or 6 a polypeptide encoded by the DNA as claimed in any of claims 1 to 3.

12. An antibody against the polypeptide of any of claims 7 to 10.

13. A oligonucleotide primer having a nucleotide sequence selected from the group consisting of the nucleotide sequences of SEQ ID NO: 7 to SEQ ID NO: 22.

14. A method of screening for modulators in known assays using constructs or of screening for interacting proteins or factors using state of the art technologies.

15. A method of screening chemical libraries comprising transformed cell lines.

16. A compound which alters or reacts with at least one epitope of the proteins and which is obtained by screening methods as claimed in claim 14 or 15.

17. The use of the antibodies according to claim 12 for diagnostic or therapeutic purposes.

18. A pharmaceutical composition comprising as an effective component an effective amount of the peptide as claimed in any of claims 7 to 10, or its salt, and a pharmaceutically acceptable carrier or diluent.
